(19) Europäisches Patentamt
European Patent Office
Office européen des brevets

(11) Veröffentlichungsnummer: **0 416 383 A2**

# (12) EUROPÄISCHE PATENTANMELDUNG

(21) Anmeldenummer: **90116202.4**

(22) Anmeldetag: **24.08.90**

(51) Int. Cl.[5]: **C07D 213/00**, A61K 31/44, C07D 213/74, C07F 7/18, C07D 213/73, C07D 213/75

(30) Priorität: **06.09.89 DE 3929507**

(43) Veröffentlichungstag der Anmeldung: **13.03.91 Patentblatt 91/11**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Fey, Peter, Dr.**
**Ursulastrasse 14**
**W-5600 Wuppertal 1(DE)**
Erfinder: **Angerbauer, Rolf, Dr.**
**Moospfad 20**
**W-5600 Wuppertal(DE)**
Erfinder: **Hübsch, Walter, Dr.**
**Am Eckbusch 43/50**
**W-5600 Wuppertal 1(DE)**
Erfinder: **Philipps, Thomas, Dr.**
**Silesiusstrasse 74**
**W-5000 Köln 80(DE)**
Erfinder: **Bischoff, Hilmar, Dr.**
**Am Rohm 78**
**W-5600 Wuppertal 1(DE)**
Erfinder: **Petzinna, Dieter, Dr. Dr.**
**Peter-Berten-Strasse 10**
**W-4000 Düsseldorf 12(DE)**
Erfinder: **Schmidt, Delf, Dr.**
**Am Eckbusch 55b**
**W-5600 Wuppertal 1(DE)**

## (54) Substituierte Amino-Pyridine.

(57) Substituierte Amino-pyridine können durch Reduktion von entsprechenden Ketonen und gegebenenfalls nachfolgender Verseifung, Cyclisierung, Hydrierung und Isomeren trennung hergestellt werden. Die substituierten Aminopyridine können als Wirkstoffe in Arzneimitteln verwendet werden.

EP 0 416 383 A2

## SUBSTITUIERTE AMINO-PYRIDINE

Die Erfindung betrifft substituierte Amin-Pyridine, Zwischenverbindungen zu ihrer Herstellung, ihre Herstellung und ihre Verwendung in Arzneimitteln.

Es is bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP 22 478; US 4 231 938].

Aus der DOS 38 01 406 und der DOS 38 01 440 sind Pyridine bzw. disubstituierte Pyridine mit inhibitorischer Wirkung auf die HMG-CoA-Reduktase bekannt.

Es wurden nun substituierte Amino-Pyridine der allgemeinen Formel (I),

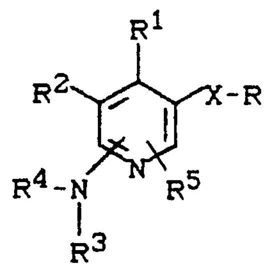

(I)

in welcher

X - für eine Gruppe der Formel $-CH_2-CH_2-$ oder $-CH=CH-$steht,

R - für eine Gruppe der Formel

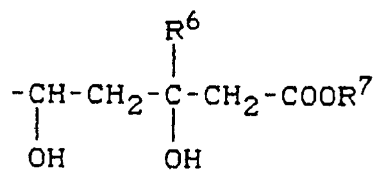

oder

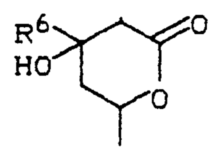

steht,

worin

$R^6$ - Wasserstoff oder geradkettiges oder verzweigtes Alkyl mit bis zu 10 Kohlenstoffatomen bedeutet und

$R^7$ - Wasserstoff oder geradkettiges oder verzweigtes Alkyl mit bis zu 10 Kohlenstoffatomen bedeutet, das durch Phenyl substituiert sein kann, oder

- Aryl mit 6 bis 10 Kohlenstoffatomen oder ein Kation bedeutet,

$R^1$ - für Aryl mit 6 bis 10 Kohlenstoffatomen steht, das gegebenenfalls bis zu 4-fach gleich oder verschieden durch geradkettiges oder verzeigtes Alkyl, Alkylthio, Alkylsulfonyl, Alkoxy oder Alkoxycarbonyl mit jeweils bis zu 8 Kohlenstoffatomen substituiert ist, die ihrerseits durch Hydroxy oder Phenyl substituiert sein können, oder durch Aryl, Aryloxy, Arylthio mit 6 bis 10 Kohlenstoffatomen, Benzyloxy, Halogen, Nitro, Cyano, Trifluormethyl, Trifluormethoxy oder Benzyloxy substituiert ist,

$R^2$ - für eine Gruppe der Formel $-CH_2-OR^8$ oder -X-R steht,

worin

$R^8$ - Wasserstoff, geradkettiges oder verzweigtes Alkyl mit bis zu 10 Kohlenstoffatomen bedeutet, das gegebenenfalls durch Hydroxy, Halogen oder Phenyl substituiert ist,

- Aryl mit 6 bis 10 Kohlenstoffatomen bedeutet, oder

- eine Gruppe der Formel $-COR^9$ bedeutet,

worin

$R^9$ - geradkettiges oder verzweigtes Alkyl mit bis zu 8 Kohlenstoffatomen oder Phenyl bedeutet,

und X und R die oben angegebene Bedeutung haben,

$R^3$ und $R^4$ gleich oder verschieden sind und

- für Wasserstoff, geradkettiges oder verzweigtes Alkyl mit bis zu 8 Kohlenstoffatomen, Phenyl oder für eine Gruppe der Formel $A-R^{10}$ steht,

worin

A - Carbonyl oder Sulfonyl bedeutet
und
$R^{10}$ - Amino oder Alkoxy mit bis zu 8 Kohlenstoffatomen bedeutet, geradkettige oder verzweigtes Alkyl mit bis zu 8 Kohlenstoffatomen oder Phenyl bedeutet, die ihrerseits durch Hydroxy, Alkoxy mit bis zu 8 Kohlenstoffatomen oder Halogen substituiert sein können, oder
- Trifluormethyl bedeutet,
oder
$R^3$ und $R^4$ gemeinsam mit dem Stickstoffatom einen gesättigten oder ungesättigten 5- bis 7-gliedrigen Heterocyclus mit bis zu 3 Heteroatomen aus oder Reihe Stickstoff, Schwefel oder Sauerstoff bilden,
und
$R^9$ - für geradkettiges oder verzweigtes Alkyl mit bis zu 10 Kohlenstoffatomen steht, oder
- für Cycloalkyl mit 3 bis 8 Kohlenstoffatomen steht
und deren Salze gefunden.

Bildet $R^7$ mit der Carboxygruppe einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_6$) und Aralkylester ($C_7$ bis $C_{10}$), bevorzugt ($C_1$-$C_4$)-Alkylester sowie Benzylester. Darüberhinaus seien die folgenden Esterreste genannt: Methylester, Ethylester, Propylester, Benzylester.

Steht $R^7$ für ein Kation, so ist bevorzugt ein physiologisch verträgliches Metall- oder Ammoniumkation gemeint. Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nichttoxische substituierte Ammoniumkationen aus Aminen wie ($C_1$-$C_4$)-Dialkylamine, ($C_1$-$C_4$)-Trialkylamine, Prokain, Dibenzylamin, N,N′-Dibenzylethylendiamin, N-Benzyl-β-phenylethylamin, N-Methylmorpholin oder N-Ethylmorpholin, 1-Ephenamin, Dihydroabietylamin, N,N′-Bis-dihydroabietylethylendiamin, N-Niederalkylpiperidin und andere Amine, die zur Bildung von Salzen verwendet werden können.

Überraschenderweise zeigen die erfindungsgemäßen substituierten Amino-Pyridine eine überlegene inhibitorische Wirkung auf die HMG-CoA-Reduktase (3-Hydroxy-3-methyl-glutaryl-Coenzym A Reduktase).

Bevorzugt sind Verbindungen der allgemeinen Formel (I),
in welcher
X - für eine Gruppe der Formel $-CH_2-CH_2-$ oder $-CH=CH-$steht,
R - für eine Gruppe der Formel

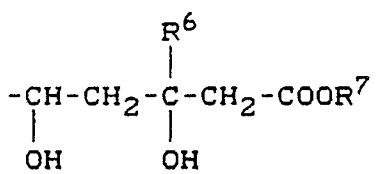 oder 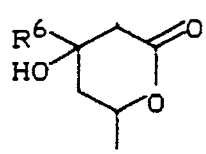 steht,

worin
$R^6$ - Wasserstoff oder geradkettiges oder verzweigtes Alkyl mit bis zu 8 Kohlenstoffatomen bedeutet
und
$R^7$- Wasserstoff oder geradkettiges oder verzweigtes Alkyl mit bis zu 8 Kohlenstoffatomen oder Benzyl bedeutet, oder
- Phenyl oder ein Kation bedeutet,
$R^1$ - für Phenyl steht, das gegebenenfalls bis zu 3-fach gleich oder verschieden durch geradkettiges oder verzweigtes Alkyl, Alkoxy oder Alkoxycarbonyl mit jeweils bis zu 6 Kohlenstoffatomen substituiert ist, die ihrerseits durch Phenyl substituiert sein können, oder
durch Phenyl, Phenoxy, Fluor, Chlor, Brom, Benzyloxy, Trifluormethyl oder Trifluormethoxy substituiert ist,
$R^2$ - für eine Gruppe der Formel $-CH_2-OR^8$ oder X-R steht,
worin
$R^8$ - Wasserstoff, geradkettiges oder verzweigtes Alkyl mit bis zu 6 Kohlenstoffatomen bedeutet, das gegebenenfalls durch Hydroxy, Fluor, Chlor oder Phenyl substituiert ist, oder
- Phenyl bedeutet, oder
- eine Gruppe der Formel $-COR^9$ bedeutet,
worin
$R^9$ - geradkettiges oder verzweigtes Alkyl mit bis zu 6 Kohlenstoffatomen bedeutet,
und X und R die oben angegebene Bedeutung haben,

$R^3$ und $R^4$ gleich oder verschieden sind und
- für Wasserstoff, geradkettiges oder verzweigtes Alkyl mit bis zu 6 Kohlenstoffatomen, Phenyl oder
- für die Gruppe der Formel $-A-R^{10}$ steht,
worin
A - Carbonyl oder Sulfonyl bedeutet
und
$R^{10}$ - geradkettiges oder verzweigtes Alkyl mit bis zu 6 Kohlenstoffatomen, Phenyl oder Trifluormethyl bedeutet
oder
$R^3$ und $R^4$ gemeinsam mit dem Stickstoffatom einen Morpholin-, Piperidin- oder Pyrrolidinring bilden,
und
$R^5$ - für geradkettiges oder verzweigtes Alkyl mit bis zu 8 Kohlenstoffatomen, Cyclopropyl, Cyclopentyl oder Cyclohexyl steht
und deren Salze.

Besonders bevorzugt sind Verbindungen der allgemeinen Formel (I),
in welcher
X - für eine Gruppe -CH=CH- steht,
R - für eine Gruppe der Formel

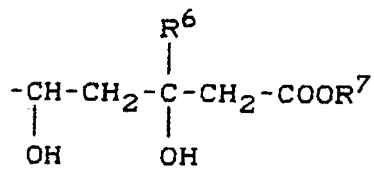 oder 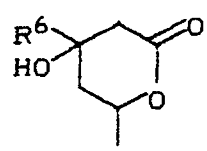 steht,

worin
$R^6$ - Wasserstoff, Methyl, Ethyl, Propyl, Isopropyl, Butyl, Isobutyl oder tert.Butyl bedeutet
und
$R^7$ - Wasserstoff, Methyl, Ethyl, Propyl, Isopropyl, Butyl, Isobutyl, tert.Butyl oder Benzyl bedeutet, oder
- ein Natrium-, Kalium-, Calcium-, Magnesium-oder Ammoniumion bedeutet,
$R^1$ - für Phenyl steht, das gegebenenfalls bis zu 2-fach gleich oder verschieden durch geradkettiges oder verzweigtes Alkyl oder Alkoxy mit jeweils bis zu 4 Kohlenstoffatomen, Benzyl, Fluor, Chlor, Phenoxy oder Benzyloxy substituiert ist,
$R^2$ - für eine Gruppe der Formel $-CH_2-OR^8$ oder -X-R steht,
worin
$R^8$ - Wasserstoff, geradkettiges oder verzweigtes Alkyl mit bis zu 4 Kohlenstoffatomen oder Phenyl bedeutet,
und X und R die oben angegebene Bedeutung haben,
$R^3$ und $R^4$ gleich oder verschieden sind und
- für Wasserstoff oder geradkettiges oder verzweigtes Alkyl mit bis zu 4 Kohlenstoffatomen oder Phenyl oder für die Gruppe der Formel $-A-R^{10}$ steht,
worin
A - Carbonyl bedeutet und
$R^{10}$ geradkettiges oder verzweigtes Alkyl mit bis zu 4 Kohlenstoffatomen bedeutet, oder
$R^3$ und $R^4$ gemeinsam mit dem Stickstoffatom einen Piperidin- oder Pyrrolidinring bilden,
$R^5$ - für geradkettiges oder verzweigtes Alkyl mit bis zu 6 Kohlenstoffatomen oder Cyclopropyl steht,
und deren Salze.

Ganz besonders bevorzugt steht $R^6$ für Wasserstoff.

Die erfindungsgemäßen substituierten Amino-Pyridine der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes R ergeben sich unterschiedliche Stereoisomere, die im folgenden näher erläutert werden sollten:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

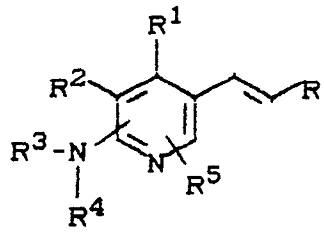

(II) E-Form

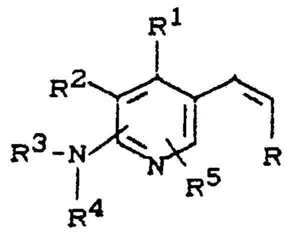

(III) Z-Form

in welcher
$R^1$, $R^2$, $R^3$, $R^4$, $R^5$ und R die oben angegebene Bedeutung haben.
Bevorzugt sind diejenigen Verbindungen der allgemeinen Formel (I) die E-konfiguriert sind (II).
b) Steht der Rest -R für eine Gruppe der Formel

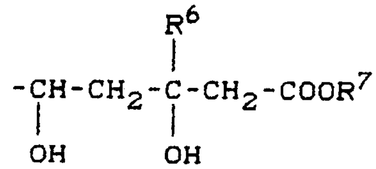

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

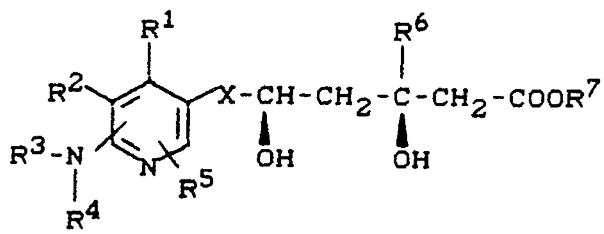

Erythro-Form (IV)

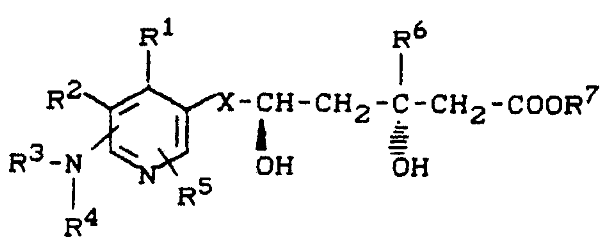

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -R- für eine Gruppe der Formel

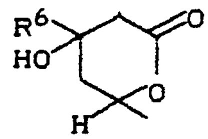

so besitzen die substituierten Amino-Pyridine mindestens zwei asymmetrishe Kohlenstoffatome, nämlich das Kohlenstoffatom, an dem die Hydroxygruppe gebunden ist, und das Kohlenstoffatom an welchem der Rest der Formel

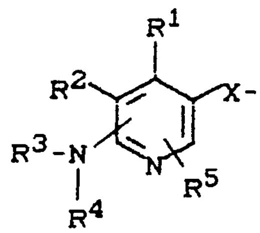

gebunden ist. Je nach der Stellung der Hydroxygruppe zur freien Valenz am Lactonring können die substituierten Amino-Pyridine als cis-Lactone (VI) oder als trans-Lactone (VII) vorliegen.

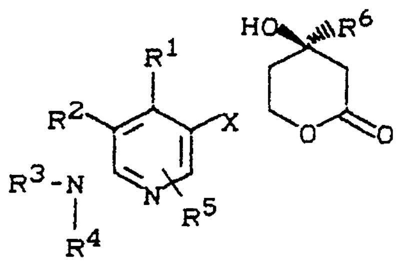

cis-Lacton (VI)

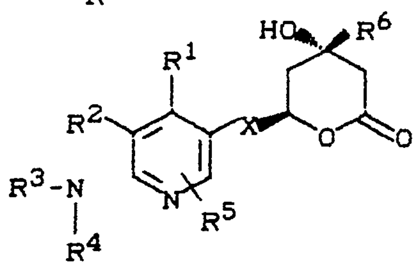

trans-Lacton (VII)

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Amino-Pyridine genannt:

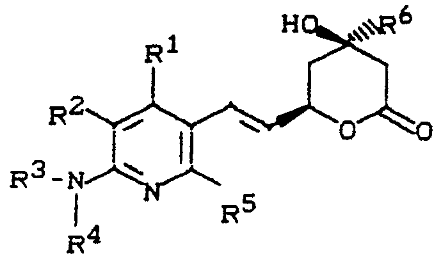

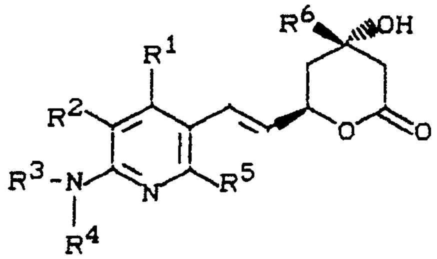

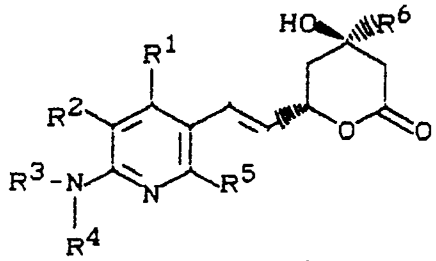

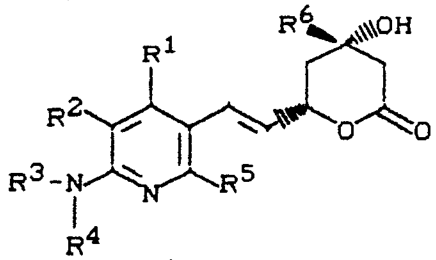

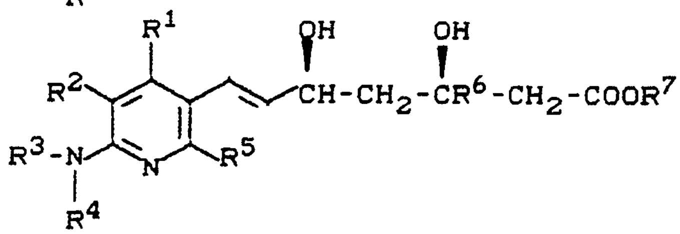

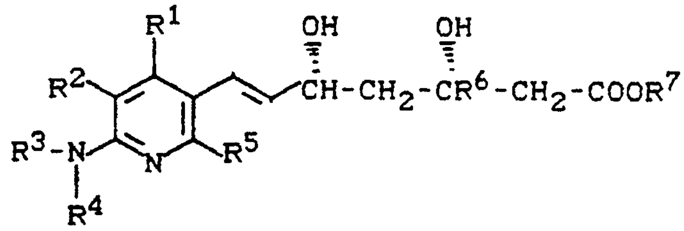

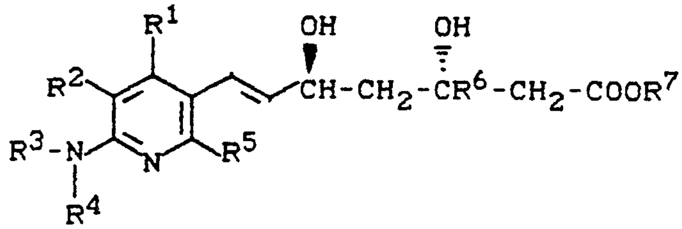

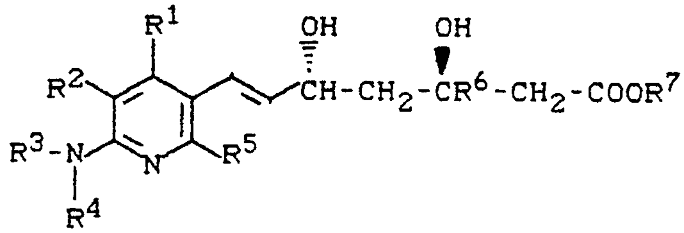

Außerdem wurde ein Verfahren zur Herstellung der substiuierten Amino-Pydridine der allgemeinen Formel (I),

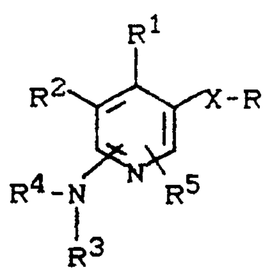

(I)

in welcher
$R^1$, $R^2$, $R^3$, $R^4$, $R^5$, X und R die oben angegebene Bedeutung haben,
gefunden, das dadurch gekennzeichnet ist, daß man
Ketone der allgemeinen Formel (VIII)

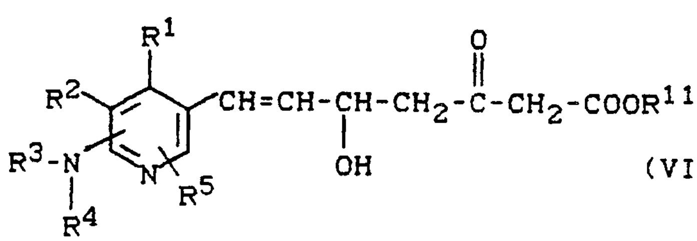

(VIII)

in welcher
$R^1$, $R^2$, $R^3$, $R^4$ und $R^5$ die oben angegebene Bedeutung haben,
und

$R^{11}$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgende Formelschema erläutert werden:

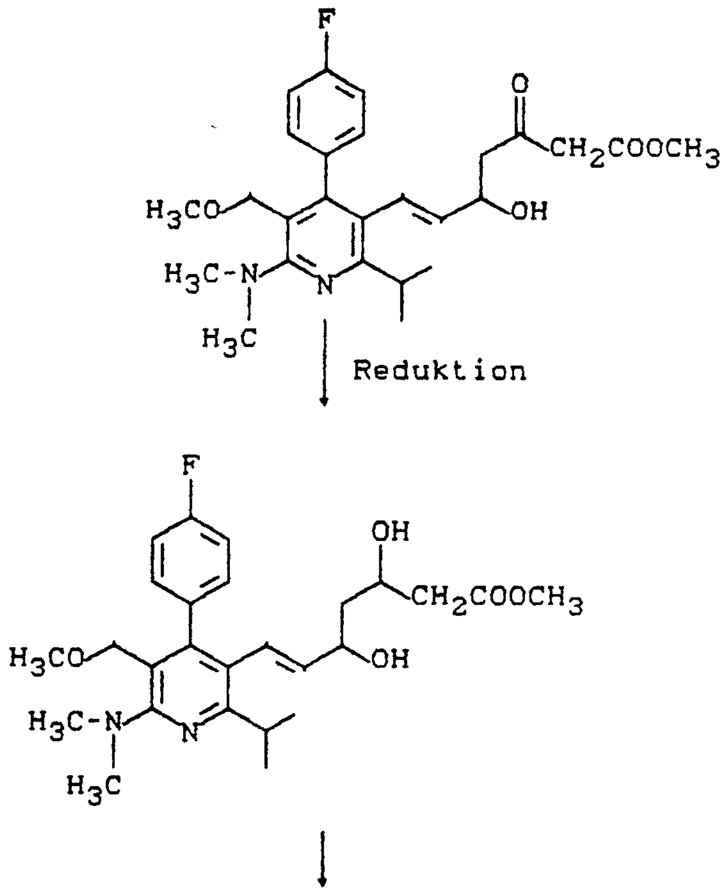

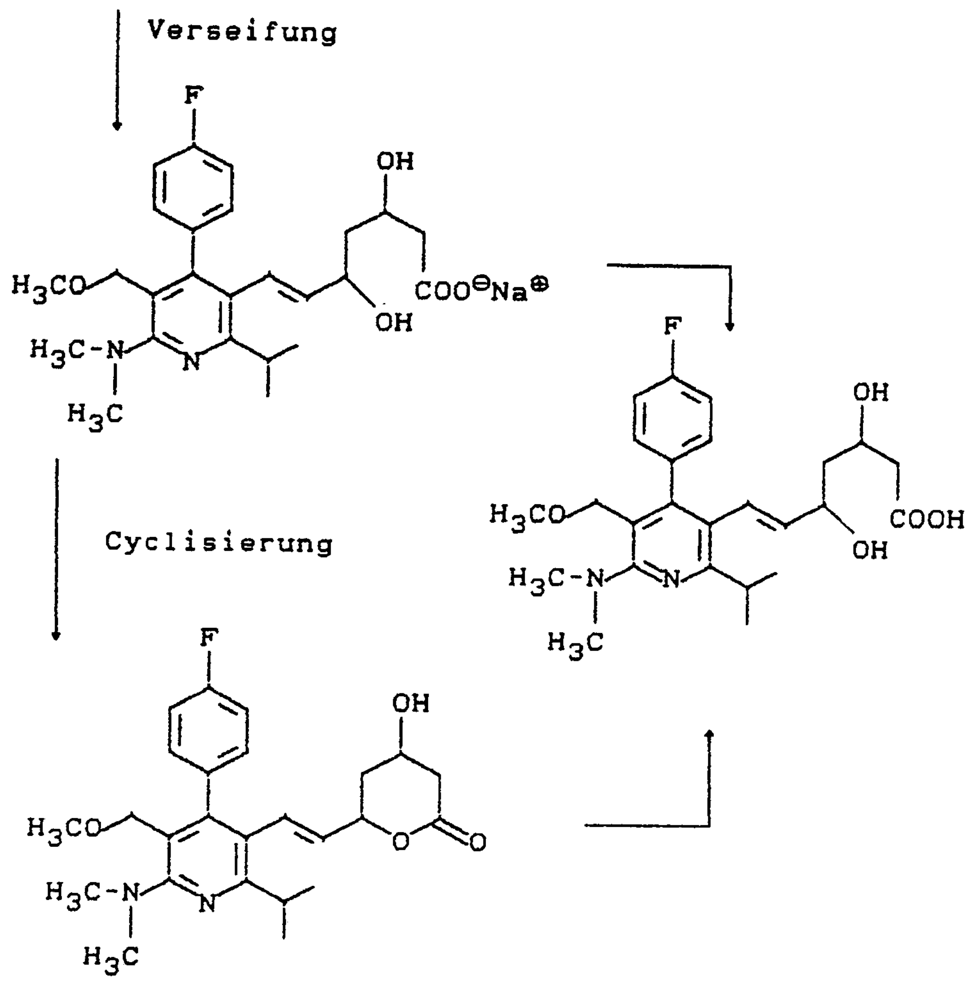


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80 °C bis +30 °C, bevorzugt von -78 °C bis 0 °C.

Das erfindungsgemäße Vorfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur

Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.
Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ia)

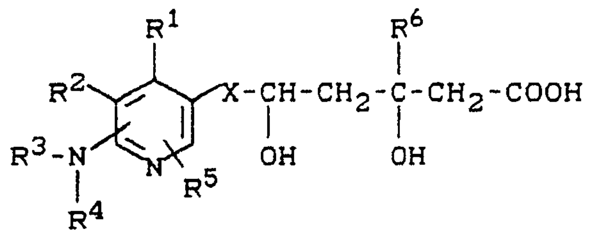

(Ia)

in welcher
$R^1$, $R^2$ $R^3$, $R^4$, $R^5$ und $R^6$ die oben angegebene Bedeutung haben.

Die Carbonsäureester im Rahmen der allgemeinen Formel (I) entsprechen der Formel (Ib)

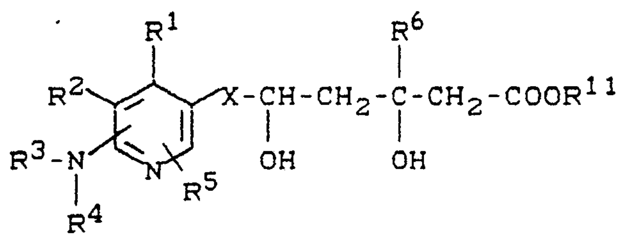

(Ib)

in welcher
$R^1$, $R^2$ $R^3$, $R^4$, $R^5$ und $R^6$ die oben angegebene Bedeutung haben,
und
$R^{11}$ - für Alkyl steht.

Die Salze der erfindungsgemäßen Verbindungen im Rahmen der allgemeinen Formel (I) entsprechen der Formel (Ic)

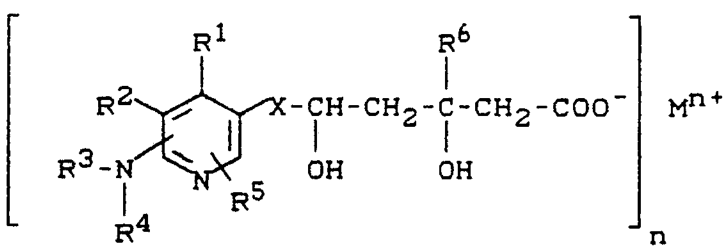

(Ic)

in welcher
$R^1$, $R^2$, $R^3$, $R^4$, $R^5$ und $R^6$ die oben angegebene Bedeutung haben,
und
$M^{n+}$ für ein Kation steht.

Die Lactone im Rahmen der allgemeinen Formel (I) entsprechen der Formel (Id)

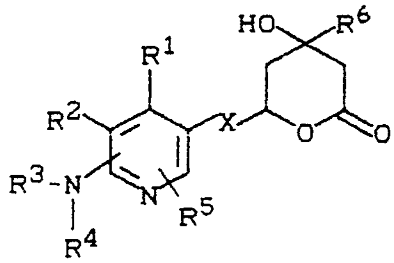

(Id)

in welcher
$R^1$, $R^2$ $R^3$, $R^4$, $R^5$ und $R^6$ die oben angegebene Bedeutung haben.

Zur Herstellung der erfindungsgemäßen Carbonsäuren der allgemeinen Formel (Ia) werden im allgemeinen die Carbonsäureester der allgemeinen Formel (Ib) oder die Lactone der allgemeinen Formel (Id) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ic) entstehen, die anschließend in einem zweiten Schritt durch Behandeln mit Säure in die freien Säuren der allgemeinen Formel (Ia) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ic) als Zwischenprodukte, die isoliert werden können. Die erfindungsgemäßen Säuren (Ia) erhält man durch Behandeln der Salze (Ic) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ia) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (Id) werden im allgemeinen die erfindungsgemäßen Carbonsäuren (Ia) nach üblichen Methoden cyclisiert. beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen, Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-Paratoluolsulfonat, N-Cyclohexyl- N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 be schrieben wird. Bevorzugt wird hierbei die Trennung der Isomeren auf der Stufe der racemisohen Lactone. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ie)

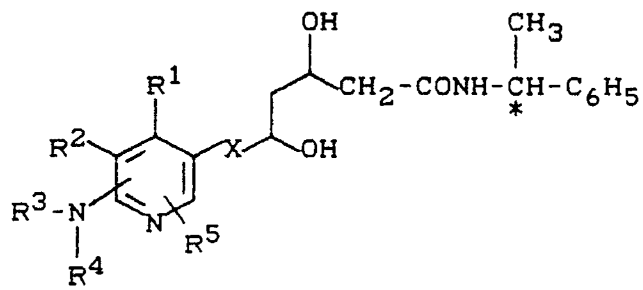

(Ie)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ia), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

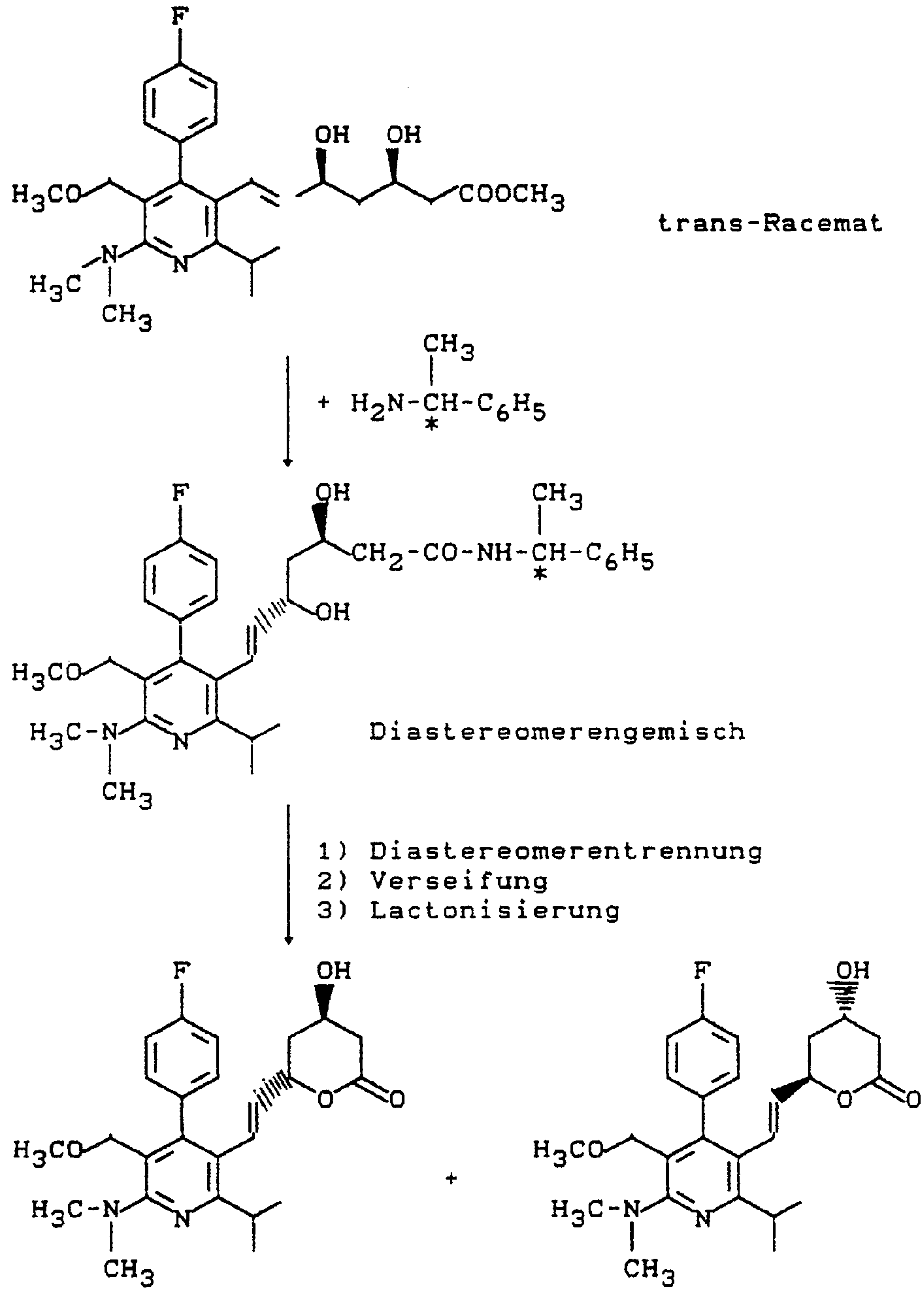


Die als Ausgangsstoffe eingesetzten Ketone (VIII) sind neu.
Es wurde ein Verfahren zur Herstellung der erfindungsgemäßen Ketone der allgemeinen Formel (VIII)

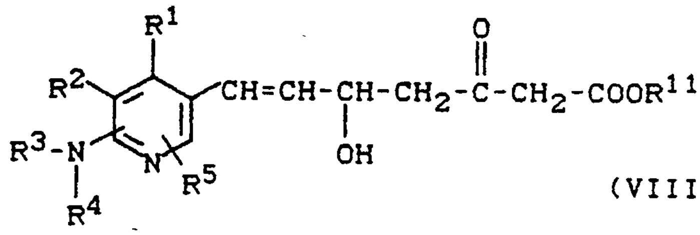

(VIII)

in welcher
$R^1$, $R^2$, $R^3$, $R^4$, $R^5$ und $R^{11}$ die oben angegebene Bedeutung haben,
gefunden, das dadurch gekennzeichnet ist, daß man
Aldehyde der allgemeinen Formel (IX)

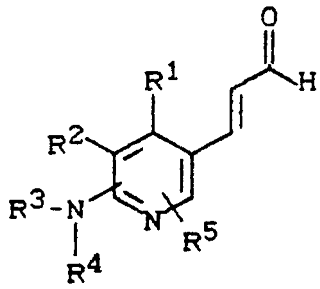

(IX)

in welcher
$R^1$, $R^2$, $R^3$, $R^4$ und $R^5$ die oben angegebene Bedeutung haben,
in inerten Lösemitteln mit Acetessigester der allgemeinen Formel (X)

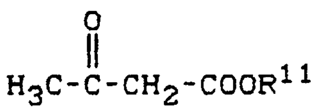

(X)

in welcher
$R^{11}$ die oben angegebene Bedeutung hat,
in Anwesenheit von Basen umsetzt.

Das erfindungsgemäße Verfahren kann beispielsweise durch folgendes Formelschema erläutert werden:

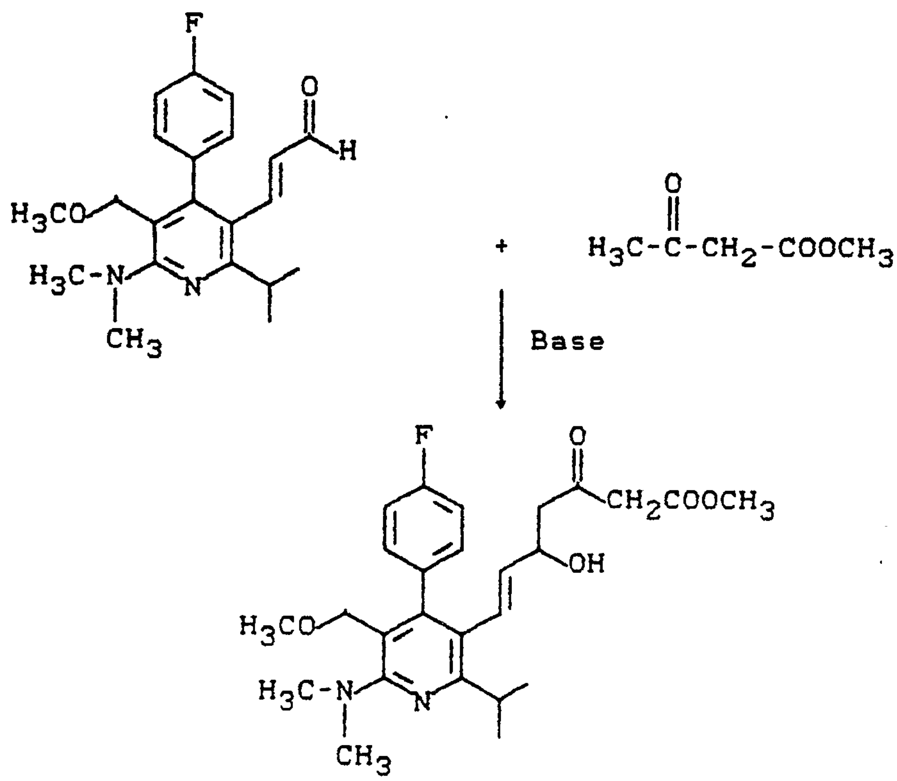


Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropylamid, Natriumamid

oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III , 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel (IX) soll im folgenden beispielhaft für die Verbindungen des Typs (If) mit $R^2$ = -X-R erläutert werden.

[A]

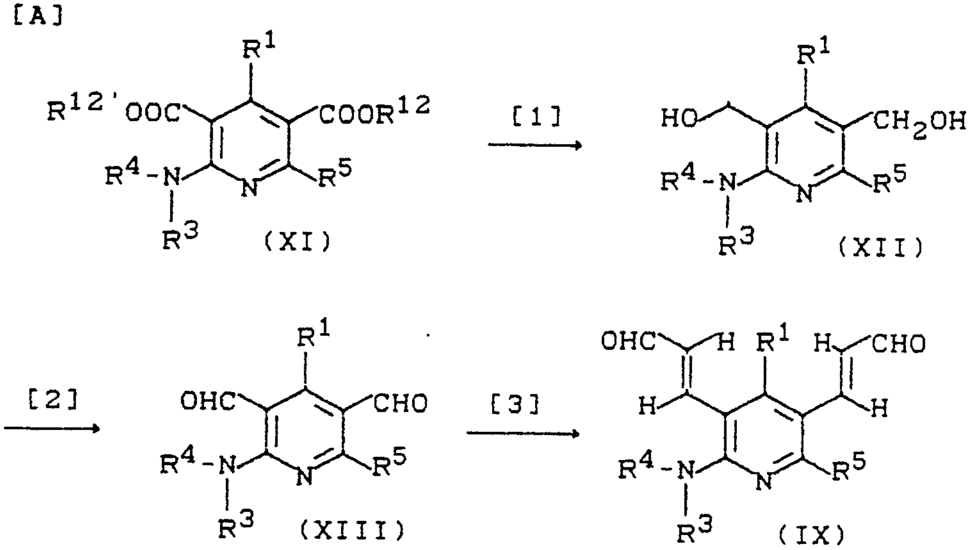

(XI) (XII) (XIII) (IX)

Hierbei werden gemäß Schema A Pyridine der Formel (XI), in welchen $R^{12}$ und $R^{12'}$ gleich oder verschieden sind und für Alkyl mit bis zu 4 Kohlenstoffatomen stehen, im ersten Schritt [1] in inerten Lösemitteln wie Ethern, beispielsweise Diethylether, Tetrahydrofuran oder Dioxan, vorzugsweise Tetrahydrofuran, mit Metall hydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70 °C bis +100 °C, vorzugsweise von -70 °C bis Raumtemperatur, bzw. von Raumtemperatur bis 70 °C je nach verwendetem Reduktionsmittel zu den Hydroxymethylverbindungen (XII) reduziert. Vorzugsweise wird die Reduktion mit Lithiumaluminiumhydrid in Tetrahydrofuran in einem Temperaturbereich von Raumtemperatur bis 80 °C durchgeführt. Die Hydroxymethylverbindungen (XII) werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0 °C bis 60 °C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (XIII) werden im dritten Schritt [3] mit Diethyl-2-(cyclohexylamino)-vinylphosphonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20 °C bis +40 °C,

vorzugsweise von -5° C bis Raumtemperatur zu den Aldehyden (IX) umgesetzt.

Die hierbei als Ausgangsstoffe eingesetzten Pyridine der Formel (XI) erhält man hierbei im allgemeinen gemäß Schema B durch Oxidation von Dihydropyridinen (XIV), die wiederum je nach der Bedeutung des Restes $R^2$ durch Variation der entsprechenden funktionellen Gruppen erhalten wurden. Die hierbei als Ausgangsstoffe eingesetzten Dihydropyridine sind teilweise neu oder bekannt und können nach bekannten Methoden, beispielsweise durch eine Knoevenagelreaktion mit N,N-Dimethyl-ethoxycarbonyl-acetamidin-hydrochlorid und (E/Z)-4-Carboxymethyl-5-(4-fluorphenyl)2-methyl-pent-4-en-3-on, hergestellt werden [vgl. außerdem EP-A 88 276, DE-A 28 47 236). Die Oxidation der Dihydropyridine (XIV) zu den Pyridinen (XI) kann beispielsweise mit Chromoxid in Eisessig in einem Temperaturbereich von -20° C bis +150° C, vorzugsweise bei Rückflußtemperatur, oder mit 2,3-Dichlor-5,6-dicyan-p-benzochinon als Oxidationsmittel in inerten Lösemitteln wie Chlorkohlenwasserstoffe, vorzugsweise Methylenchlorid in einem Temperaturbereich von 0° C bis +100° C, vorzugsweise bei Raumtemperatur durchgeführt werden.

[B]

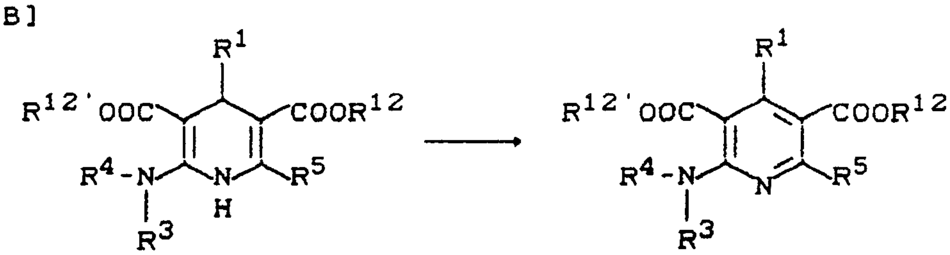

(XIV) (XI)

Im Fall, daß in den Ausgangsverbindungen der allgemeinen Formel (IX) $R^2$ für den Rest $-CH_2-OR^8$ steht, worin $R^8$ die oben angegebene Bedeutung hat, verläuft die Herstellung in Analogie zu denen unter Schema A aufgeführten Schritten [2] und [3]. In einem vorgelagerten Schritt werden aber gemäß Schema [C] die Verbindungen der Formel (XV) hergestellt.

[C]

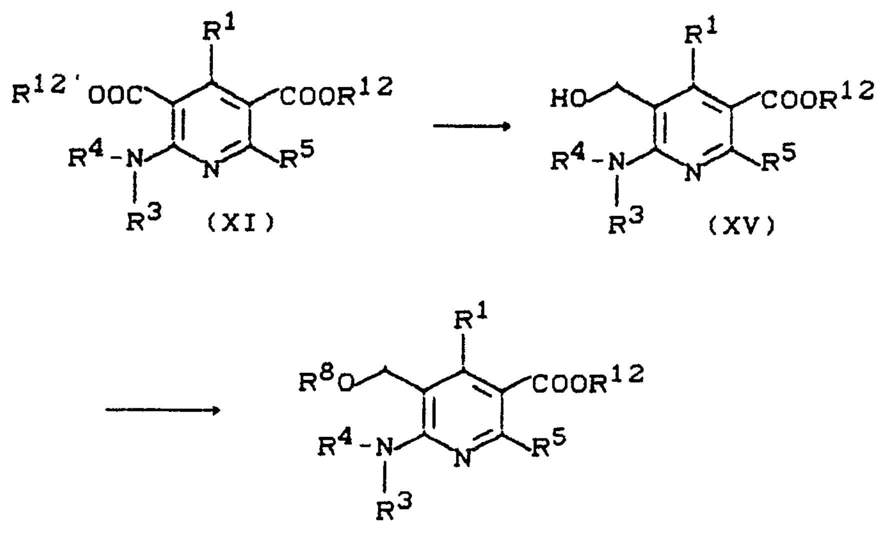

(XI) (XV)

(XVI)

Die Pyridine (XI), die wie oben beschrieben, aus den entsprechenden Dihydropyridinen durch Oxidation hergestellt werden, können durch geeignete Reduktionsmittel, wie beispielsweise Lithiumaluminiumhydrid, Diisobutylaluminiumhydrid oder Natrium-bis-(2-methoxy-ethoxy)-dihydroaluminat in inerten Lösemitteln, wie beispielsweise Tetrahydrofuran, zu den Pyridinen (XV) reduziert werden.

Die Pyridine (XV) können nach bekannten Methoden zu den Pyridinen (XVI) umgesetzt werden, beispielsweise durch Reaktion mit einem Alkyl- oder Benzylhalogenid in Gegenwart einer Base wie beispielsweise Natriumhydrid oder beispielsweise durch Umsetzung mit einem Trialkylsilylhalogenid oder

einem Säurehalogenid in Gegenwart einer Base wie Imidazol, Pyridin oder Triethylamin. Die Hydroxygruppe der Pyridine (XV) kann nach bekannten Methoden in eine Abgangsgruppe überführt werden, z.B. durch Umsetzung mit Trifluormethansulfonsäureanhydrid, Thionylchlorid oder Methansulfonsäurechlorid in Gegenwart einer Base. Die Abgangsgruppe kann dann nach bekannten Methoden gegen Nucleophile ausgetauscht werden.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methyl-glutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atherosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G.C. Ness et al., Archives of Biochemistry and Biophysics 197 , 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000 g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78 °C eingefroren und gelagert ( = Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37 °C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $K_xH_y$ -Phosphat pH 7,2, 20 µl Enzympräparation und 56 nMol 3-Hydroxy-3-methyl-glutaryl Coenzym A (Glutaryl-3-$^{14}$C) 100 000 dpm.

Nach Inkubation von 60 Minuten bei 37 °C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Starke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin

können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

Ethoxycarbonyl-ethanimidsäureethylester-hydrochlorid

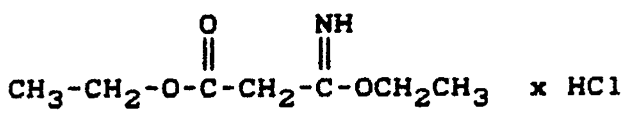

Bei 0-10 °C leitet man in eine Lösung von 678 g (6 mol) Cyanessigsäureethylester in 400 ml Ethanol und 3 l Ether 7 h Salzsäuregas, läßt über Nacht bei 25 °C stehen, saugt den ausgefallenen Feststoff ab und trocknet im Vakuum.
Ausbeute: 742 g (63% der Theorie)
Schmp.: 108-110 °C

### Beispiel 2

Ethoxycarbonyl-ethanimidsäureethylester

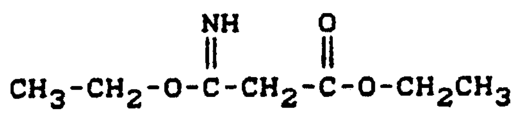

140 g (0,72 mol) der Verbindung aus Beispiel 1 werden portionsweise unter Rühren in eine Mischung von 128 g (0,93 mol) Kaliumcarbonat, 750 ml Eiswasser und 400 ml Ether gegeben. Die Phasen werden getrennt, die wäßrige Phase mit Ether gewaschen, die Etherphasen vereinigt, getrocknet ($Na_2SO_4$) und das Lösemittel bei 30 °C am Rotationsverdampfer entfernt. Die Destillation des Rückstandes bei 80-82 °C / 12 mm liefert 107,3 g Produkt.
Ausbeute: 93,7% der Theorie

Beispiel 3

N,N-Dimethyl-ethoxycarbonyl-acetamidin-hydrochlorid

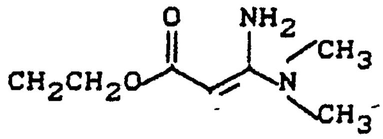

x HCl

In 300 ml Ethanol werden 107,3 g (0,67 mol) der Verbindung aus Beispiel 2 und 55 g (0,67 mol) Dimethylaminhydrochlorid über Nacht unter Rückfluß erhitzt. Das Lösemittel wird abdestilliert und der Rückstand mit Aceton verrieben.
Ausbeute: 94,5g (72,5% der Theorie)
Schmp.: 110-112° C

Beispiel 4

(E/Z)-5-(4-fluorphenyl)-2-methyl-4-methoxycarbonyl-pent-4-en-3-on

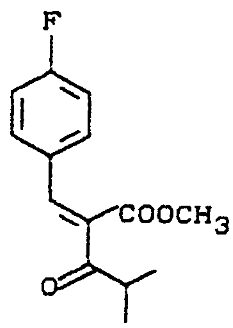

62 g (0,5 Mol) 4-Fluorbenzaldehyd und 79 g (0,5 mol) Isobutyrylessigsäuremethylester werden in 300 ml Isopropanol vorgelegt und mit einem Gemisch aus 2,81 ml (28 mmol) Piperidin und 1,66 ml (29 mmol) Essigsäure in 40 ml Isopropanol versetzt. Man läßt 48 Stunden bei Raumtemperatur rühren, engt im Vakuum ein und destilliert den Rückstand im Hochvakuum.
Kp 4 mm: 150-152° C
Ausbeute: 110,9 g (84% der Theorie)

Beispiel 5

1,4-Dihydro-6-dimethylamino-4-(4-fluorphenyl)-2-isopropyl-pyridin-3,5-dicarbonsäure-5-ethyl-3-methylester

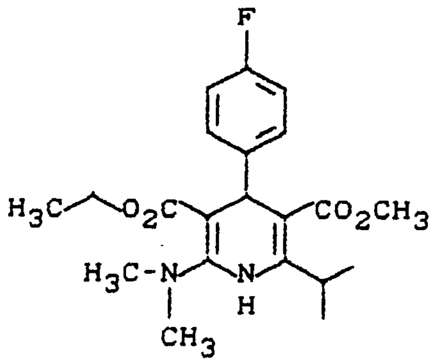

94,5 g (0,486 mol) der Verbindung aus Beispiel 3, 121,5 g (0,486 mol) der Verbindung aus Beispiel 4 und 53,4 ml (0,486 mol) N-Methyl-morpholin werden über Nacht bei 120 °C gerührt, nach dem Abkühlen in Methylenchlorid gelöst, mit Wasser gewaschen, getrocknet (Natriumsulfat) eingeengt und aus Methanol umkristallisiert.
Ausbeute: 140,0 g (73,8% der Theorie)
Schmp.: 154-156 °C

Beispiel 6

6-Dimethylamino-4-(4-fluorphenyl)-2-isopropyl-pyridin-3,5-dicarbonsäure-5-ethyl-3-methylester

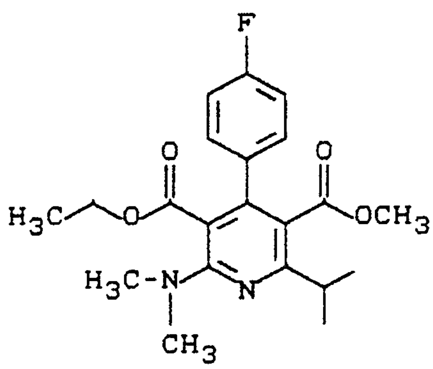

Man gibt in eine Lösung von 65,4 g (0,17mol) der Verbindung aus Beispiel 5 in 1 l Methylenchlorid bei 25 °C 49,5 g (0,22 mol) 2,3-Dichlor-5,6-dicyan-p-benzochinon und rührt über Nacht. Der ausgefallene Feststoff wird abfiltriert und mit Methylenchlorid gewaschen. Die vereinigten Methylenchloridphasen werden über Kieselgel (70-230 mesh) filtriert.
Ausbeute: 56,2 g (86% der Theorie)
$^1$H-NMR ($CDCl_3$): $\delta$ (ppm) = 0,9 (tr, 3H); 1,25 (d, 6H); 3,0 (sept, 1H); 3,1 (s, 6H); 3,4 (s, 3H); 3,9 (q, 2H); 6,9 - 7,3 (m, 4H).

Beispiel 7

6-Dimethylamino-4-(4-fluorphenyl)-3-hydroxymethyl-2-isopropyl-5-carbonsäureethylester

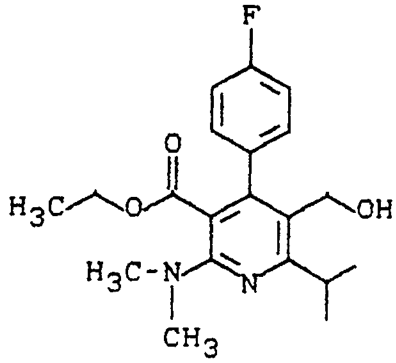

Unter Stickstoff gibt man zu einer Lösung von 56,2 g (0,145 mol) der Verbindung aus Beispiel 6 in 250 ml Tetrahydrofuran bei 25 °C 85 ml (0,29 mol) einer 3,5-molaren Lösung von Natrium-bis-(2-methoxyethoxy)dihydroaluminat in Toluol und rührt bei 50 °C über Nacht. Man hydrolysiert vorsichtig mit einer Lösung von 100 g Kaliumnatriumtartrat in 200 ml Wasser, trennt die Phasen, wäscht die wäßrige Phase mit Ether, wäscht die vereinigten organischen Phasen mit Wasser, trocknet über Natriumsulfat (und chromatographiert an einer Kieselgelsäule mit Petrolether/Essigester 5:1.
Ausbeute: 9,1 g (17,4% der Theorie)
$^1$H-NMR ($CDCl_3$): $\delta$ (ppm) = 0,9 (t, 3H); 1,3 (d, 6H); 3,1 (s, 6H); 3,4 (sept, 1H); 3,85 (q, 2H); 4,3 (s, 2H); 7,0-7,25 (m, 4H).
als Nebenprodukt erhält man:

Beispiel 8

3,5-Dihydroxymethyl-6-dimethylamino-4-(4-fluorphenyl)-2-isopropyl-pyridin

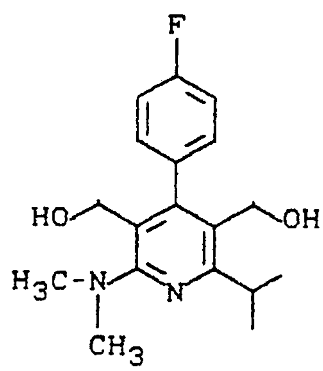

Ausbeute: 5,6 g (12,1% der Theorie)
$^1$H-NMR ($CDCl_3$): $\delta$ (ppm) = 1,3 (d, 6H); 2,85 (s, 6H); 3,4 (sept, 1H); 4,3 (s, 2H); 4,4 (s, 2H); 7,1-7,2 (m, 4H).

Beispiel 9

6-Dimethylamino-4-(4-fluorphenyl)-2-isopropyl-pyridin-3,5-dicarbaldehyd

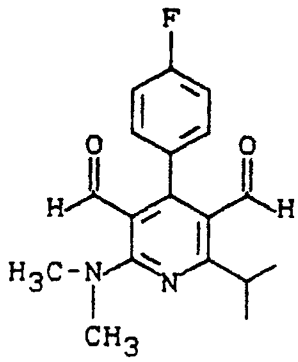

In eine Lösung von 5,64 g (17,6 mmol) der Verbindung aus Beispiel 8 in 1 l Methylenchlorid werden 9,1 g (42,3 mmol) Pyridiniumchlorochromat eingetragen und über Nacht bei 25° C gerührt. Die Suspension wird über Kieselgur und anschließend über Kieselgel filtriert.
Ausbeute: 1,84 g (33,3% der Theorie)
$^1$H-NMR ($CDCl_3$): $\delta$ (ppm) = 1,25 (d,6H); 3,2 (s, 6H); 4,1 (sept, 1H); 7,1 - 7,5 (m, 4H); 9,3 (s, 1H); 9,5 (s, 1H).

Beispiel 10

(E,E)-6-Dimethylamino-3,5-di-(prop-2-en-1-al-3-yl)-4-(4-fluorphenyl)-2-isopropyl-pyridin

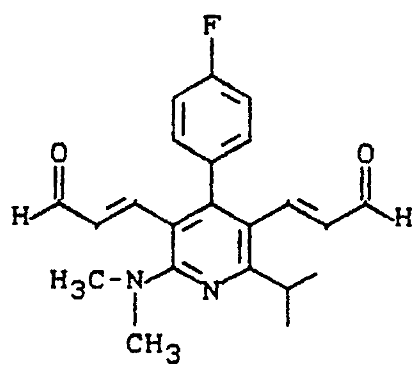

Unter Stickstoff tropft man zu einer Suspension von 344 mg (14,3 mmol) Natriumhydrid in 20 ml trockenem Tetrahydrofuran bei -5° C 4,5 g (17,2 mmol) Diethyl-2-(cyclohexylamino)-vinylphosphonat, gelöst in 12 ml trockenem Tetrahydrofuran. Nach 30 min werden bei derselben Temperatur 1,8 g (5,7 mmol) der Verbindung aus Beispiel 9 in 20 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Magnesiumsulfat getrocknet. Nach Einengen im Vakuum wird der Rückstand in 70 ml Toluol aufgenommen, mit einer Lösung von 3,8 g (49 mmol) Oxalsäure-Dihydrat in 50 ml Wasser versetzt und 30 min zum Rückfluß erhitzt. Nach Abkühlen auf Raumtemperatur werden die Phasen getrennt, die organische Phase mit gesättigter Natriumchlorid-Lösung gewaschen, über Magnesiumsulfat getrocknet und im Vakuum eingeengt. Der Rückstand wird an einer Säule (100 g Kieselgel 70-230 mesh ⌀ 3,5 cm, mit Methylenchlorid) chromatographiert.
Ausbeute: 1,42 g (67,6% der Theorie)
$^1$H-NMR ($CDCl_3$): $\delta$ (ppm) = 1,3 (d, 6H); 3,0 (s, 6H); 3,35 (sept, 1H); 5,85 (dd, 1H); 6,1 (dd, 1H); 7,0 - 7,3 (m, 6H); 9,35 (m, 2H).

Beispiel 11

6-Dimethylamino-3,5-bis-(methyl-(E)-5-hydroxy-3-oxo-hept-6-enoat-7-yl)-4-(4-fluorphenyl)-2-isopropyl-pyridin

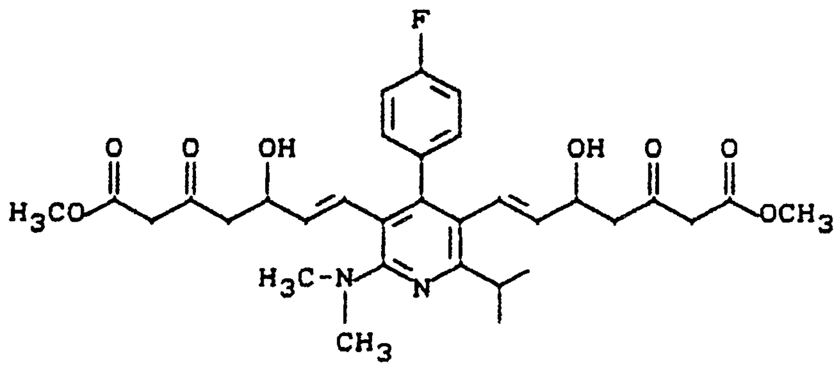

Unter Stickstoff tropft man zu einer Suspension von 583 mg (24,3 mmol) Natriumhydrid in 20 ml trockenem Tetrahydrofuran bei -5° C 2,57 ml (23,8 mmol) Acetessigsäuremethylester. Nach 15 min werden bei derselben Temperatur 17 ml (24,3 mmol) 15%iges Butyllithium in n-Hexan Zugetropft und 15 min nachgerührt. Anschließend werden 1,39 g (3,8 mmol) der Verbindung aus Beispiel 10 gelöst in 20 ml trockenem Tetrahydrofuran zugetropft und 30 min bei -5° C nachgerührt. Die Reaktionslösung wird mit 100 ml wäßriger Ammoniumchloridlösung versetzt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Magnesiumsulfat getrocknet und im Vakuum eingeengt.
Rohausbeute: 3,04 g (>100% der Theorie)

Beispiel 12

6-Dimethylamino-3,5-bis-(methyl-erythro-(E)-3,5-dihydroxy-hept-6-enoat-7-yl)-4-(4-fluorphenyl)-2-isopropyl-pyridin

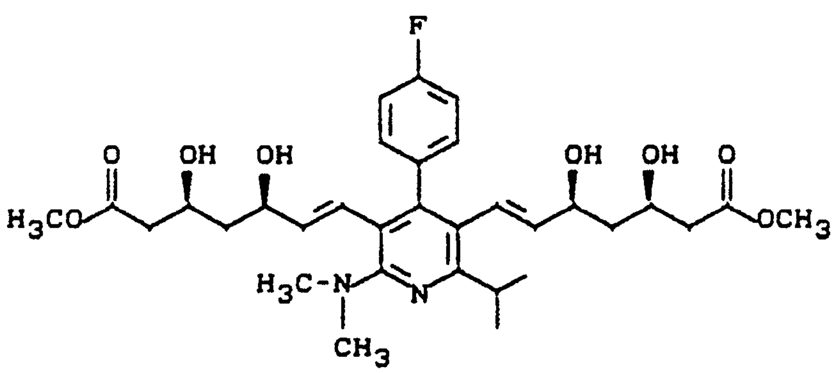

Zu einer Lösung von 3,0 g (5,0 mmol) der Verbindung aus Beispiel 11 in 80 ml trockenem Tetrahydrofuran gibt man bei Raumtemperatur 11 ml (11 mmol) 1 M Triethylboran-Lösung in Tetrahydrofuran, leitet während 5 min Luft durch die Lösung und kühlt auf -30° C Innentemperatur ab. Es werden 416 mg (11 mmol) Natriumborhydrid und langsam 19,3 ml Methanol zugegeben, 30 min bei -30° C gerührt und dann mit einem Gemisch von 34 ml 30%igem Wasserstoffperoxid und 70 ml Wasser versetzt. Die Temperatur läßt man dabei bis 0° C ansteigen und rührt noch 30 min nach. Die Mischung wird dreimal mit je 100 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Kieselgelsäule (230-400 mesh) mit Essigester/Petrolether 2:1 chromatographiert.

Ausbeute: 860 mg (38% der Theorie)
$^1$H-NMR ($CDCl_3$): δ (ppm) = 1,2 (d, 6H); 1,25-1,6 (m, 4H); 2,4 (m, 4H); 2,9 (s, 6H); 3,25 (sept, 1H); 3,7 (2s, 6H); 4,1 (m, 2H); 4,3 (m, 2H); 5,2 (dd, 1H); 5,5 (dd, 1H); 6,2 (dd, 2H); 7,0 (m, 4H).

Beispiel 13

6-Dimethylamino-4-(4-fluorphenyl)-2-isopropyl-3-methoxymethyl-pyridin-5-carbonsäure-ethylester

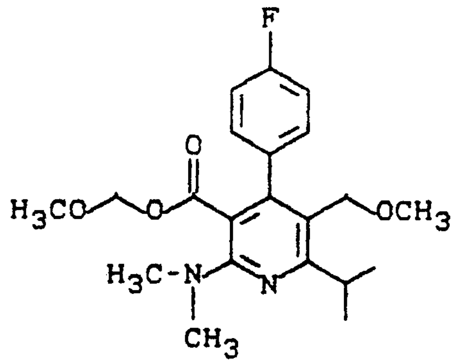

Zu einer Lösung von 1,95 g (5,42 mmol) der Verbindung aus Beispiel 7 in 30 ml Tetrahydrofuran gibt man unter Stickstoff bei 0 °C 143 mg (5,96 mmol) Natriumhydrid. Nach 30 min addiert man 1,0 ml (16,2 mmol) Methyliodid und rührt bei 25 °C über Nacht. Dann hydrolysiert man vorsichtig mit Eiswasser, wäscht 3 mal mit Ether und erhält nach dem Trocknen 1,88 g Öl.
Ausbeute: 92,5% der Theorie
$^1$H-NMR ($CDCl_3$): δ (ppm) = 0,9 (t, 3H); 1,25 (d, 6H); 3,0 (s, 6H); 3,1 (s, 3H); 3,3 (sept, 1H); 3,85 (q, 2H); 4,0 (s, 2H); 7,0 -7,2 (m, 4H).

Beispiel 14

6-Dimethylamino-4-(4-fluorphenyl)-5-hydroxymethyl-2-isopropyl-3-methoxymethyl-pyridin

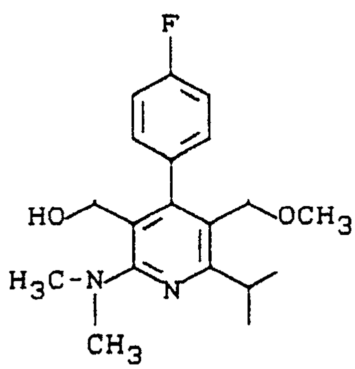

Unter Stickstoff gibt man zu einer Lösung von 1,83 g (4,9 mmol) der Verbindung aus Beispiel 13 in 10 ml trockenem Tetrahydrofuran bei 25 °C 2,8 ml (9,8 mmol) einer 3,5 molaren Lösung von Natrium-bis-(2-methoxyethoxy)-dihydroaluminat in Toluol und rührt über Nacht bei 60 °C. Nach Abkühlen auf 20 °C tropft man vorsichtig eine Lösung von 10 g Kalium-Natriumtartrat in 20 ml Wasser zu und extrahiert dreimal mit je 100 ml Essigester. Die vereinigten organischen Phasen werden einmal mit gesättigter Natriumchlorid-Lösung gewaschen, über Natriumsulfat getrocknet und im Vakum eingeengt.

Rohausbeute: 1,96 g (>100% der Theorie)
$^1$H-NMR ($CDCl_3$): δ (ppm) = 1,2 (d, 6H); 2,8 (s, 6H), 3,1 (s, 3H); 3,25 (sept, 1H); 3.95 (s, 2H); 4.3 Cs, 2H); 7,0-7,2 (m, 4H).

Beispiel 15

6-Dimethylamino-4-(4-fluorphenyl)-2-isopropyl-3-methoxymethyl-pyridin-5-carbaldehyd

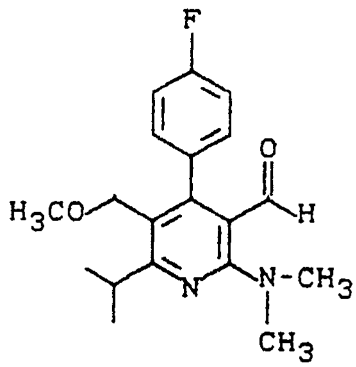

Analog zu Beispiel 9 erhält man aus 1,86 g (5,6 mmol) der Verbindung aus Beispiel 14 540 mg Öl.
Ausbeute: 35,6% der Theorie
$^1$H-NMR ($CDCl_3$): δ (ppm) = 1,3 (d, 6H); 3,1 (s, 6H); 3,2 (s, 3H); 3,3 (spet, 1H); 4,0 (s, 2H); 7,1 - 7,4 (m, 4H); 9,4 (s, 1H).

Beispiel 16

(E)-3-[6-Dimethylamino-4-(4-fluorphenyl)-2-isopropyl-3-methoxymethyl-pyrid-5-yl]-prop-2-enal

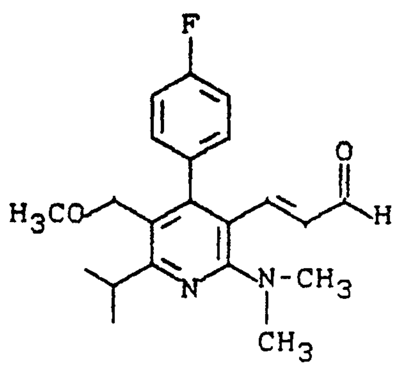

Analog zu Beispiel 10 erhält man aus 520 mg (1,58 mmol) der Verbindung aus Beispiel 15 470 mg Öl.
Ausbeute; 83,7% der Theorie
$^1$H-NMR ($CDCl_3$); δ (ppm) = 1,3 (d, 6H); 2,9 (s, 6H); 3,15 (s, 3H); 3,3 (sept, 1H); 3,95 (s, 2H); 6,1 (dd, 1H); 7,0 -7,3 (m, 5H); 9,3 (d, 1H).

Beispiel 17

Methyl-(E)-7-[6-Dimethylamino-4-(4-fluorphenyl)-2-isopropyl-3-methoxymethyl-pyrid-5-yl]-5-hydroxy-3-oxo-hept-6-enoat

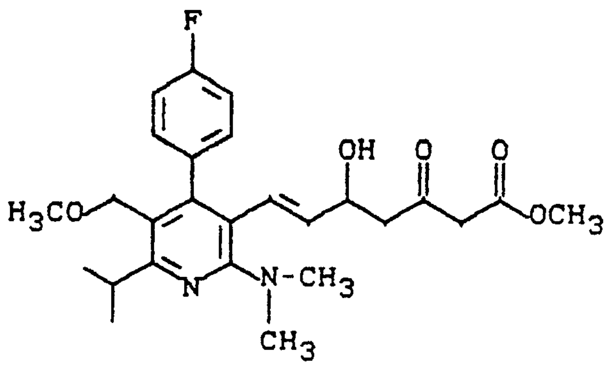

Analog zu Beispiel 11 erhält man aus 420 mg (1,18 mmol) der Verbindung aus Beispiel 16 780 mg Rohprodukt.
Ausbeute: >100% der Theorie
$^1$H-NMR ($CDCl_3$): δ (ppm) = 1,3 (d, 6H); 1,5 (m, 2H); 2,5 (m, 2H); 2,85 (s, 6H); 3,15 (s, 3H); 3,3 (sept, 1H); 3,7 (s, 3H); 3,95 (s, 2H); 4,45 (m, 1H); 5,45 (dd, 1H); 6,2 (d, 1H); 7,1 (m, 4H).

Beispiel 18

Methyl-erythro-(E)-7-[dimethylamino-4-(4-fluorphenyl)-2-isopropyl-3-methoxymethyl-pyrid-5-yl]-3,5-dihydroxy-hept-6-enoat

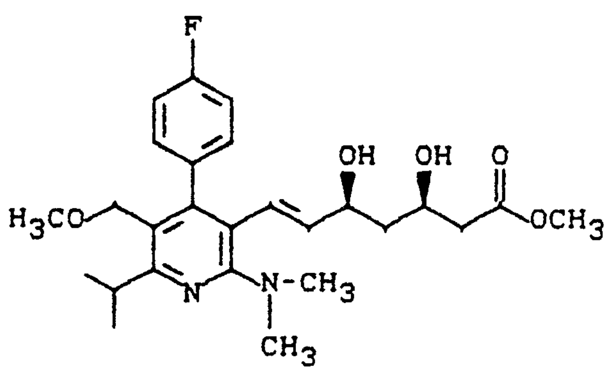

Analog zu Beispiel 12 erhält man aus 720 mg (1,18 mmol) der Verbindung aus Beispiel 17 217 mg Öl.
Ausbeute: 38,7% der Theorie
$^1$H-NMR ($CDCl_3$): δ (ppm) = 1,25 (d, 6H); 1,5 (m, 2H); 2,4 (m, 2H); 2,9 (s, 6H); 3,1 (s, 3H); 3,3 (sept, 1H); 3,7 (s, 3H); 3,95 (s, 2H); 4,1 (m, 1H); 4,25 (m, 1H); 5,45 (dd, 1H); 6,2 (d, 1H); 7,0 - 7,2 (m, 4H).

Beispiel 19

3-(tert.-Butyldimethylsilyloxymethyl)-6-dimethylamino-4-(4-fluorphenyl)-2-isopropyl-pyridin-5-carbonsäureethyl ester

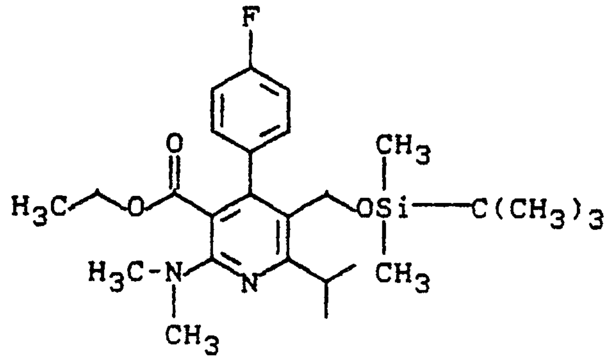

Zu einer Lösung von 5,03 g (14 mmol) der Verbindung aus Beispiel 7 in 50 ml Dimethylformamid gibt man bei Raumtemperatur 2,32 g (15,4 mmol) tert.Butyldimethylsilylchlorid, 1,9 g (28 mmol) Imidazol und 0,05 g 4-Dimethylaminopyridin. Es wird über Nacht bei Raumtemperatur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Natriumsulfat getrocknet und im Vakuum eingeengt.

Rohausbeute: 7,58 g (>100% der Theorie)

$^1$H-NMR ($CDCl_3$): $\delta$ (ppm) = 0,0 (s, 6H); 0,8 (s, 9H); 1,25 (d, 6H); 3,0 (s, 6H); 3,3 (sept, 1H); 3,8 (q, 2H); 4,2 (s, 2H); 6,9 - 7,3 (m, 4H).

Beispiel 20

3-(tert.Butyldimethylsilyloxymethyl)-6-dimethylamino-4-(4-fluorphenyl)-5-hydroxymethyl-2-isopropyl-pyridin

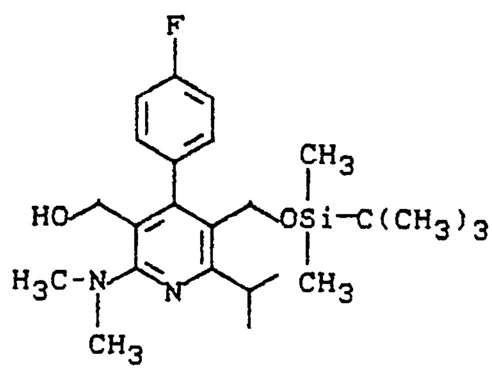

Analog zu Beispiel 14 erhält man aus 7,52 g (14 mmol) der Verbindung aus Beispiel 19 7,19 g Rohprodukt.

Ausbeute: >100% der Theorie

$^1$H-NMR ($CDCl_3$): $\delta$ (ppm) = - 0,1 (s, 6H); 0,8 (s, 9H); 1,3 (s, 6H); 2,85 (s, 6H); 3,35 (sept. 1H); 4,25 (s, 2H); 4,4 (s, 2H); 7,0 - 7,2 (m, 4H).

Beispiel 21

3-(tert.Butyldimethylsilyloxymethyl)-6-dimethylamino-4-(4-fluorphenyl)-2-isopropyl-5-methoxymethyl-pyridin

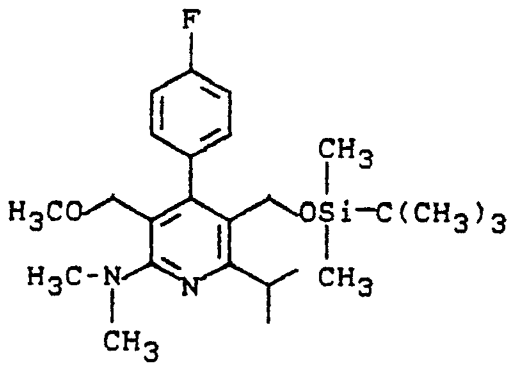

Analog zu Beispiel 13 erhält man aus 7,1 g (14 mmol) der Verbindung aus Beispiel 20 3,96 g Feststoff. Ausbeute: 63,2% der Theorie

$^{1}$H-NMR ($CDCl_3$): $\delta$ (ppm) = 0,0 (s, 6H); 0,9 (s, 9H); 1,35 (d, 6H); 3,1 (s, 6H); 3,15 (s, 3H); 3,45 (sept, 1H); 4,0 (s, 2H); 4,3 (s, 2H); 7,1 - 7,5 (m, 4H).

Beispiel 22

6-Dimethylamino-4-(4-fluorphenyl )-3-hydroxymethyl-2-isopropyl-5-methoxymethyl-pyridin

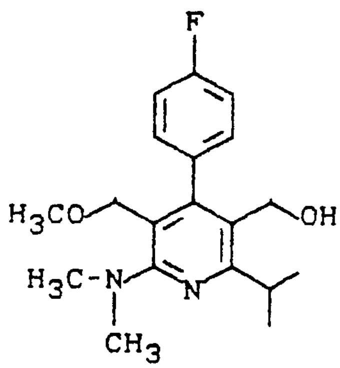

3,9 g (8,75 mmol) der Verbindung aus Beispiel 21 und 8,75 ml (8,75 mmol) einer 1 molaren Lösung von Tetrabutylammoniumfluorid in Tetrahydrofuran werden 2,5 h in 20 ml Tetrahydrofuran bei 25 °C gerührt. Nach Einengen im Vakuum wird in Essigester gelöst, 3 mal mit 10%iger Schwefelsäure und anschließend mit gesättigter Natriumhydrogencarbonatlösung und gesättigter Kochsalzlösung gewaschen. Die wäßrige Phase wird auf pH 9 eingestellt und mit Methylenchlorid gewaschen. Nach Trocknen und Einengen der vereinigten organischen Phasen erhält man 6,49 g Öl.

Rohausbeute: >100% der Theorie

Beispiel 23

6-Dimethylamino-4-(4-fluorphenyl)-2-isopropyl-5-methoxymethyl-pyridin-3-carbaldehyd

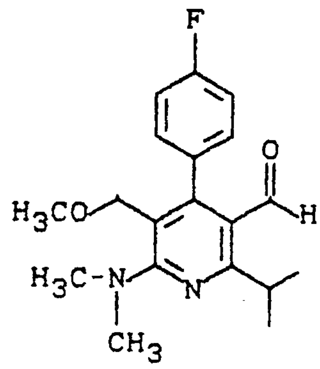

Analog zu Beispiel 9 erhält man aus 6,49 g der Verbindung aus Beispiel 22 270 mg Feststoff.
Ausbeute: 9,3% der Theorie
$^1$H-NMR ($CDCl_3$): δ (ppm) = 1,25 (d, 6H); 3,1 (s, 3H); 3,25 (s, 6H); 3,8 (s, 2H); 4,1 (sept, 1H); 7,05 - 7,35 (m, 4H); 9,5 (s, 1H).

Beispiel 24

(E)-3-(6-Dimethylamino-4-(4-fluorphenyl)-2-isopropyl-5-methoxymethyl-pyrid-3-yl]-prop-2-enal

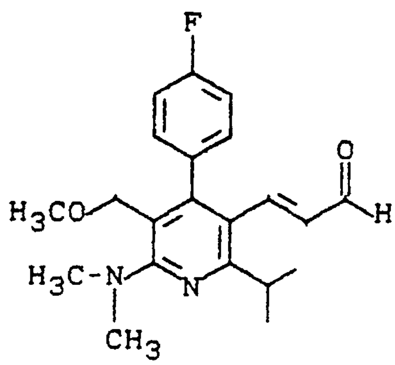

Analog zu Beispiel 10 erhält man aus 250 mg (0,76 mmol) der Verbindung aus Beispiel 23 350 mg Rohprodukt.
Ausbeute: >100% der Theorie
$^1$H-NMR ($CDCl_3$): δ (ppm) = 1,3 (d, 6H); 3,1 (s, 3H); 3,15 (s, 6H); 3,35 (sept, 1H); 3,85 (s, 2H); 5,75 (dd, 1H); 7,0 -7,35 (m, 5H); 9,3 (d, 1H).

Beispiel 25

Methyl-(E)-[6-dimethylamino-4-(4-fluorphenyl )-2-isopropyl-5-methoxymethyl-pyrid-3-yl]-5-hydroxy-3-oxo-hept-6-enoat

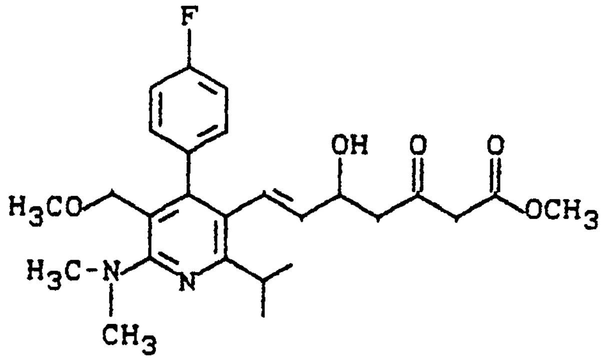

Analog zu Beispiel 17 erhält man aus 310 mg (0,67 mmol) der Verbindung aus Beispiel 24 440 mg Rohprodukt.
Ausbeute: >100% der Theorie

Beispiel 26

Methyl-erythro-(E)-7-[Dimethylamino-4-(4-fluorphenyl)-2-isopropyl-5-methoxymethyl-pyrid-5-yl]-3,5-dihydroxy-hept-6-enoat

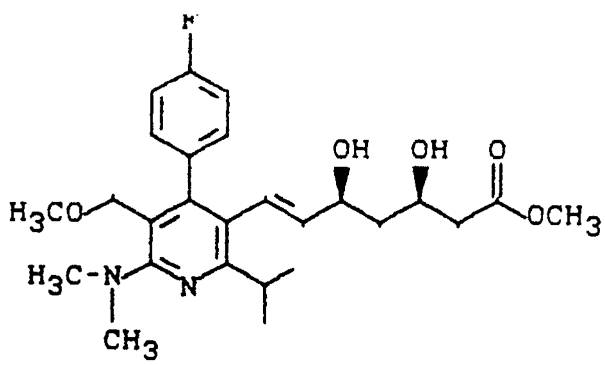

Analog zu Beispiel 12 erhält man aus 400 mg (0,61 mmol) der Verbindung aus Beispiel 25 60 mg Öl.
Ausbeute: 20,7% der Theorie
$^1$H-NMR ($CDCl_3$): $\delta$ (ppm) = 1,1 - 1,6 (m, 8H); 2,45 (m, 2H); 3,0 (s, 6H); 3,1 (s, 3H); 3,25 (sept., 1H); 3,7 (s, 3H); 3,9 (s, 2H); 4,05 (m, 1H); 4,25 (m, 1H); 5,2 (dd, 1H); 6,3 (d, 1H); 6,9-7,3 (m, 4H).

Beispiel 27

2-Amino-3-ethoxycarbonyl-4(4-fluorophenyl)-5-methoxy-carbonyl-1,4-dihydro-pyridin

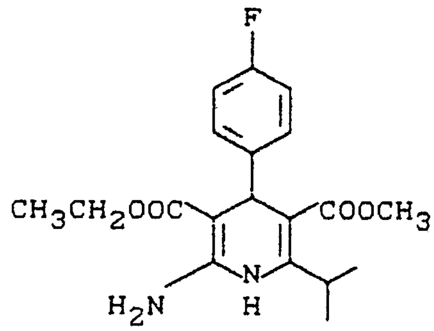

66 g (0,4 mol) Amidinoessigsäure-ethylester-hydrochlorid (H. Meyer, F. Bossert, H. Horstmann, Liebigs Ann. Chem. 1977 , 1895) werden mit 100 g (0,4 mol) der Verbindung aus Beispiel 4 und 44 ml (0,4 mol) Triethylamin über Nacht am Rückfluß gekocht. Nach dem Einengen wird der Rückstand mit Petrolether/Essigester 2:1 über 2 kg Kieselgel filtriert.
Ausbeute: 109,7 g (76 % farbl. Kristalle, Schmp. 161 °C aus Ether/Petrolether).

Beispiel 28

2-Amino-3-ethoxycarbonyl-4(4-fluorphenyl)-6-isopropyl-5-methoxycarbonyl-pyridin

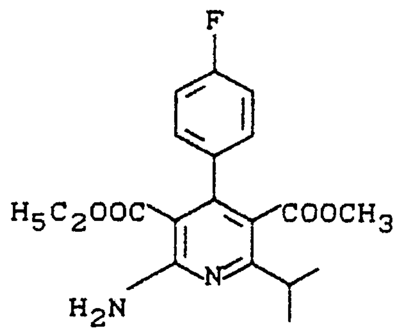

36,2 g (6,1 Mol) der Verbindung aus Beispiel 27 werden in 1 l Methylenchlorid mit 22,7 g (0,1 mol) 2,3-Dichlor-5,6-dicyano-p-benzochinon 1 h bei Raumtemperatur gerührt. Man filtriert über 1,5 kg Kieselgel 230-400 mesh, wäscht mit Petrolether/Essigsäure (2:1) nach, engt die Filtrate ein und rührt den Rückstand in Ether/Petrolether aus.
Ausbeute: 31,6 g (88 %) farbl. Kristalle vom Schmp. 141 °C.

Beispiel 29

2-Amino-4(4-fluorphenyl)-3-hydroymethyl-6-isopropyl-5-methoxycarbonyl-pyridin

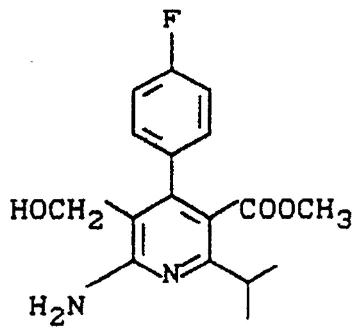

In 100 ml (0,35 mol) einer (3,5 M) toluolischen Red-Al-Lösung in 1 l THF tropft man bei 25-35° C eine Lösung von 63 g (0,175 mol) der Verbindung aus Beispiel 28 in 700 ml THF und rührt noch 1 h bei 30° C.

Dann werden 2 l Wasser vorsichtig zugegeben, zweimal mit Essigester extrahiert, die organischen Phasen mit gesättigter Kochsalzlösung gewaschen, getrocknet und eingeengt. Der Rückstand wird mit Petrolethern Essigester (1:1) über 400 g Kieselgel 230-400 mesh filtriert. Das eingeengte Filtrat ergibt nach Behandeln mit Ether/Petrolether 45-29 (81 %) farblose Kristalle vom Schmp. 137° C.

Beispiel 30

2-Amino-4(4-fluorphenyl)-6-isopropyl-5-methoxycarbonyl-3-methoxymethyl-pyridin

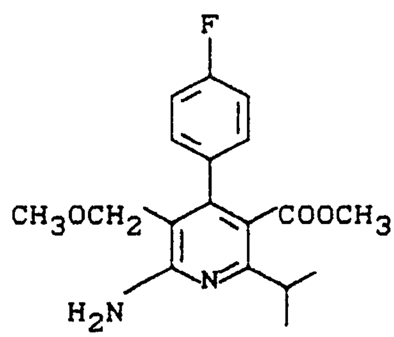

Zu einer Suspension von 2,3 g (77 mmol) 80 %igem Natriumhydrid in 150 ml THF p.a. tropft man eine Lösung von 22,2 g (70 mmol) der Verbindung aus Beispiel 29 in 150 ml THF und rührt noch 60 min bei Raumtemperatur.

Dann werden 9.95 g (70 mmol) Methyliodid zugetropft und 3 h bei Raumtemperatur gerührt. Man zersetzt mit Wasser, extrahiert zweimal mit Essigester, wäscht die org. Phasen mit ges. NaCl-Lösung, trocknet über Natriumsulfat und engt ein. Nach Chromatographie über 450 g Kieselgel 230-400 mesh (Säulendurchmesser 6 cm) mit Petrolether/Essigester (2:1) erhält man 19,0 g (82 96) farbl. Kristalle vom Schmp. 84° C.

Beispiel 31

2-Amino-4-(4-fluorphenyl)-5-hydroxymethyl-6-isopropyl-3-methoxycarbonyl-pyridin

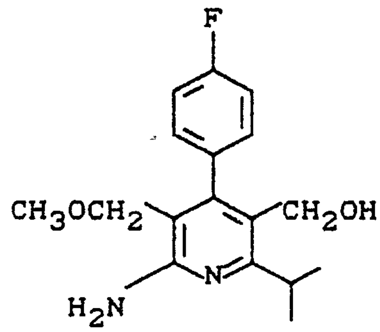

Zu einer Lösung von 23,1 g (70 mmol) der Verbindung des Beispiels 30 in 1 l Toluol tropft man bei -78 °C bis -75 °C unter Argon 140 ml (210 mmol) einer 1,5 M Lösung von Diisobutylaluminiumhydrid und rührt 1 h bei dieser Temperatur. Während man erwärmen läßt, werden 40 ml Wasser und 50 ml Essigester zugetropft, Kieselgur zugesetzt, abgesaugt und mit Essigester nachgewaschen. Die wäßrige Phase wird mit Essigester extrahiert, die vereinten org. Phasen mit ges. NaCl-Lösung gewaschen und über Natriumsulfat getrocknet. Man engt zur Trockne ein und rührt den Rückstand in Ether aus.
Ausbeute: 16,1 g (76 %) farbl. Kristalle vom Schmp. 152 °C.

Beispiel 32

2-Amino-4(4-fluorphenyl)-5-formyl-6-isopropyl-3-methoxymethyl-pyridin

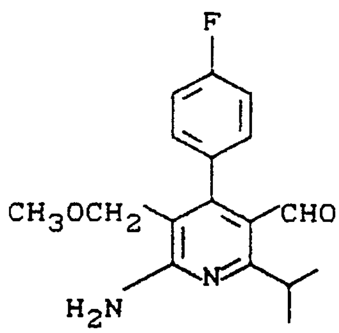

Zu einer Suspension von 16,1 g (53 mmol) der Verbindung aus Beispiel 31 in 500 ml Toluol gibt man 138,3 g (1,59 mol) aktivierten Braunstein und rührt 3 h bei 75 °C. Es wird heiß abgesaugt, der Rückstand mit 250 ml Essigester ausgekocht und nochmals mit Essigester gewaschen. Die vereinigten Filtrate werden über 400 g Kieselgel 230-400 mesh (Säulendurchmesser 6 cm) mit Petrolether/Essigester (3:1) chromatographiert.
Ausbeute: 9,5 g (59 %) farbl. Kristalle vom Schmp. 159 °C

Beispiel 33

(E)-3-[2-Amino-4(4-fluorphenyl)-6-isopropyl-3-methoxy-methyl-pyrid-5-yl]prop-2-enal

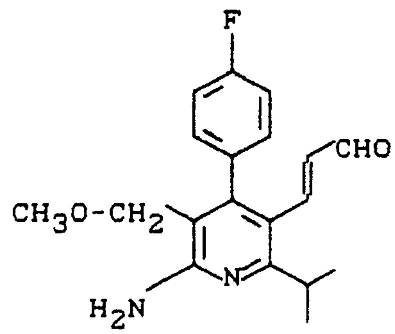

Zu 2,2 g (70,9 mmol) 80 % Natriumhydrid in 100 ml THF, tropft man bei 0-5 °C unter Argon 9,5 g (31,5 mmol) Verbindung aus Beispiel 32 in 100 ml THF rührt 10 min. bei dieser Temperatur und kocht 1 h am Rückfluß. Man versetzt mit Wasser, extrahiert zweimal mit Essigester, wäscht die vereinigten org. Phasen mit ges. NaCl-Lösung und engt ein. Der Rückstand wird in 100 ml THF und 500 ml 1N Salzsäure 1 h bei Raumtemperatur gerührt. Dann neutralisiert man mit gesättigter Natriumhydrogencarbo nat-Lösung, extrahiert zweimal mit Essigester, engt ein und chromatographiert über 400 g Kieselgel mit Petrolether/Essigester (3:1) bis (2:4).
Ausbeute: 2,4 g (23 %) gelbl. Kristalle vom Schmp. 81 °C.

Beispiel 34

Methyl-erythro-(E)-7-[2-amino-4-(4-fluorphenyl)-6-isopropyl-3-methoxymethyl-pyrid-5-yl]-3,5-dihydroxy-hept-6-enoat

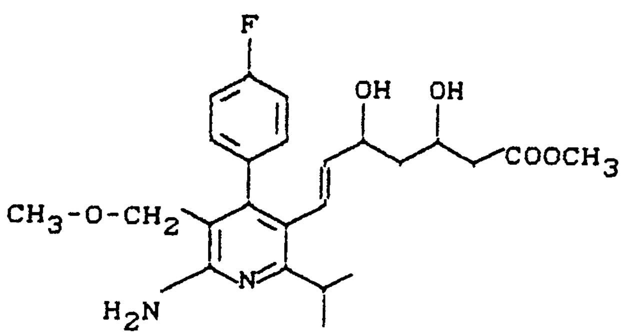

Unter Argon tropft man zu einer Suspension von 0,55 g (18,3 mmol) 80 %igem Natriumhydrid in 20 ml THF p.a. bei 0-5 °C 1,2 ml (11 mmol) Acetessigsäuremethylester und rührt 15 min nach. Dann werden 13,4 ml (22 mmol) 15 %iges Butyllithium in Hexan zugetropft, 15 min bei 0-5 °C nachgerührt und schließlich bei derselben Temperatur eine Lösung von 1,8 g (5,5 mmol) der Verbindung aus Beispiel 33 in 40 ml THF zugetropft. Nach 30 min Rühren bei Raumtemperatur tropft man 2,5 ml (43,2 mmol) Essigsäure in 40 ml Wasser zu, extrahiert zweimal mit Essigester, wäscht die vereinigten org. Phasen mit ges. NaCl-Lösung, trocknet über Natriumsulfat und engt zur Trockne ein. Der so erhaltene Rückstand (2,5 g) wird unter Argon in 35 ml Tetrahydrofuran (THF) p.a. gelöst, mit 6,6 ml einer 1 M Triethylboranlösung in THF versetzt und 5 min Luft durch die Lösung geleitet. Bei -78 °C gibt man 0,25 g (6,6 mmol) Natriumborhydrid und dann tropfenweise bei -75 °C 5,5 ml Methanol zu.

Man läßt erwärmen und gibt dabei 18,3 ml 30 %iges $H_2O_2$ und 40 ml Wasser zu, extrahiert 2 mal mit Essigester, wäscht die vereinigten org. Phasen mit ges. NaCl-Lösung und trocknet über Natriumsulfat. Chromatographie des Rückstandes (50 g Kieselgel 230-400 mesh, Säulendurchmesser 3 cm) mit Petrolether/Essigester (1:1) ergibt 1,1 g (45 % ) gelbliches Öl.
$^1$H-NMR ($CDCl_3$): δ (ppm): 1,22 (m, 6H); 1,4 (m, 2H), 2,43 (m, 2H), 3,2 (s+m, 4H), 2,7 (b, 1H); 3,5 (b, 1H), 3,72 (s, 3H) 4,1 (m+s, 3H) 4,25 (m, 1H); 4,95 (b, 2H) 5,17 (dd, 1H), 6,22 (d, 1H), 7,05 (m, 4H).

Beispiel 35

Methyl-erythro (E)-7-[2-acetylamino-4-(4-fluorphenyl)-6-isopropyl-3-methoxymethyl-pyrid-5-yl-3,5-diacetoxy-hept-6-enoat

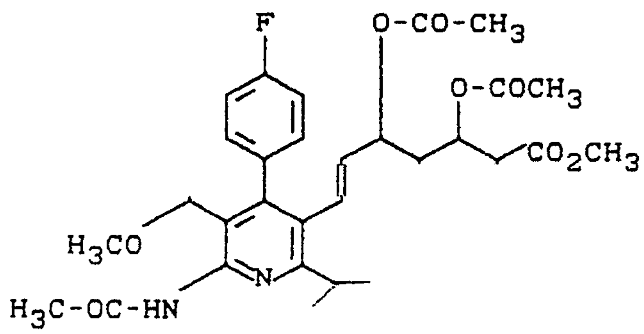

In eine Lösung von 260 mg (0,6 mmol) der Verbindung aus Beispiel 34 in 10 ml Dichlormethan gibt man 0,3 ml Pyridin, 0,25 ml Acetylchlorid und 15 mg 4-Dimethylaminopyridin und rührt 1 Tag bei Raumtemperatur. Dann wird mit Wasser versetzt, mit gesättigter Hydrogencarbonat- und Kochsalzlösung gewaschen, eingeengt und der Rückstand an 25 g Kieselgel 230-400 mesh mit Petrolether/Essigester (2:1) bis (1:1) chromatographiert.
Ausbeute: 33 mg (10 %) gelbliches Öl
DCI-MS: 573 (M + H, 100 %)

Beispiel 36

1,4-Dihydro-4-(4-fluorphenyl)-2-isopropyl-6-pyrrolidino-pyridin-3,5-dicarbonsäure-5-ethyl-3-methylester

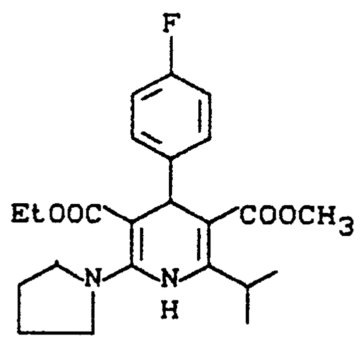

14,4 g (50 mmol) der Verbindung am Beispiel 4 und 11 g (50 mmol) 2-Amino-2-pyrrolidino-prop-2-en-carbonsäureethylester Hydrochlorid werden in 20 ml Ethanol gelöst und nach Zugabe von 6,9 ml (50 mmol) Triethylamin wird über Nacht unter Rückfluß erhitzt. Nach dem Abkühlen wird eingeengt und über Kieselgel chromatographiert (Laufmittel Essigester/Petrolether 2:8).
Ausbeute: 14,02 g (67,4 % d. Theorie)

Beispiel 37

4-(4-Fluorphenyl)-2-isopropyl-6-pyrolidino-pyridin-3,5-dicarbonsäure-5-ethyl-3-methylester

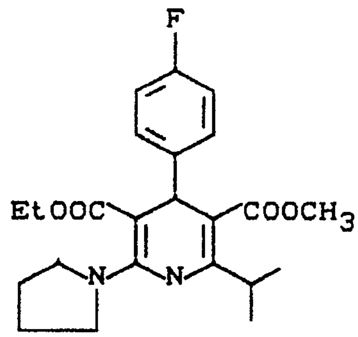

Analog zu Beispiel 6 werden aus 14 g der Verbindung aus Beispiel 36 9,2 g (66,3 d.Th.) erhalten.
$^{1}$H-NMR ($CDCl_3$): δ (ppm) = 0,9 (t, 3H); 1,26 (d, 6H); 1,95 (m, 4H); 3,07 (sept., 1H); 3,43 (s, 3H): 3,52 (m, 4H); 3,88 (q, 2H); 6,9-7,3 (m, 4H).

Beispiel 38

4-(4-Fluorphenyl)-3-hydroxymethyl-2-isopropyl-6-pyrrolidino-pyridin-5-carbonsäureethylester

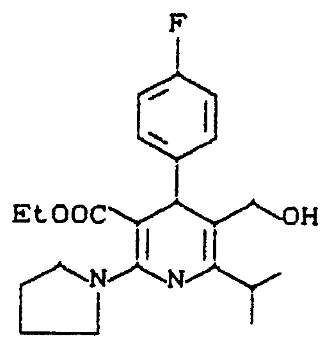

Analog zu Beispiel 7 werden aus 9,2 g der Verbindung aus Beispiel 37 6,93 g (80,9 % d.Th.) erhalten.
$^{1}$H-NMR ($CDCl_3$): δ (ppm) = 0,9 (t, 3H); 1,28 (d, 6H); 1,92 (m, 4H); 3,37 (sept., 1H); 3,51 (m, 4H); 3,85 (q, 2H); 4,31 (d, 2H); 7,0-7,3 (m, 4H).

Beispiel 39

Methyl-erythro-(E)-7-[4-(4-fluorphenyl)-2-isopropyl-5-methoxymethyl-6-pyrrolidino-pyrid-3-yl]-3,5-dihydroxy-hept-6-enoat

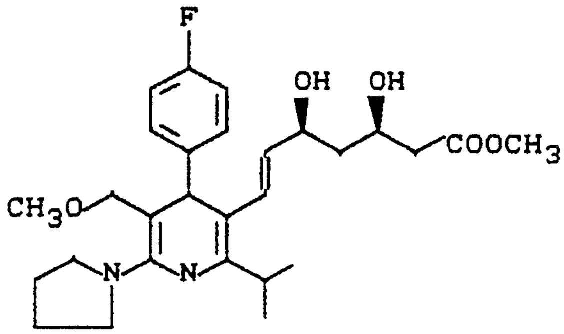

Aus der Verbindung aus Beispiel 38 wurde, in Analogie zu den Reaktionen aus den Beispielen 19, 20, 21, 22, 9, 10, 11, 12 das Beispiel 39 hergestellt.

Beispiel 40

Methyl-erythro-(E)-7-[4-(4-fluorphenyl)-2-isopropyl-3-methoxymethyl-6-pyrrolidino-pyrid-5-yl]-3,5-dihydroxy-hept-6-enoat

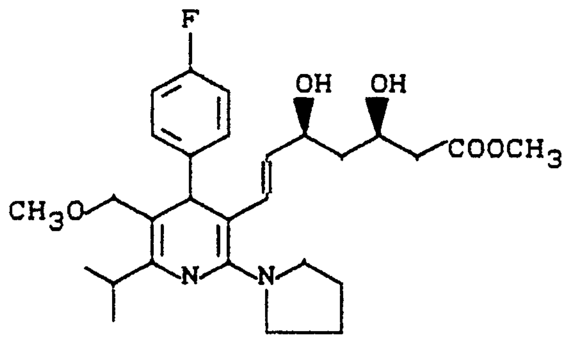

Aus der Verbindung aus Beispiel 38 wurde, in Analogie zu den Reaktionen aus den Beispielen 13, 14, 9, 10, 11, 12 das Beispiel 40 hergestellt.

Beispiel 41

1,4-Dihydro-4-(4-fluorphenyl)-2-isopropyl-6-piperidino-pyridin-3,5-dicarbonsäure-5-ethyl-3-methylester

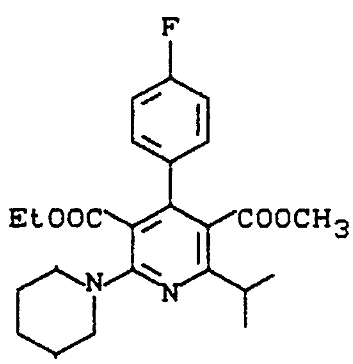

In Analogie zu Beispiel 36 werden aus 14,4 g (50 mmol) der Verbindung aus Beispiel 4 und 11,7 g (50 mmol) 2-Amino-2-piperidino-prop-2-en-carbonsäureethylester 15,1 g (70 % d.Th.) erhalten.

Beispiel 42

4-(4-Fluorphenyl)-2-isopropyl-6-piperidino-pyridin-3,5-dicarbonsäure-5-ethyl-3-methylester

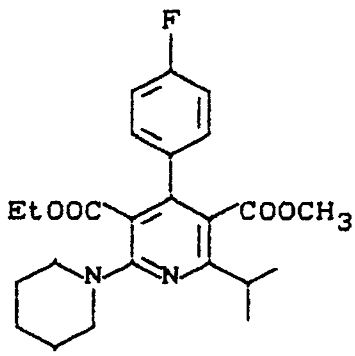

Die Herstellung erfolgt in Analogie zu Beispiel 6.
$^1$H-NMR ($CDCl_3$): δ (ppm) = 0,97 (t, 3H); 1,25 (d, 6H); 1,65 (m, 6H); 3,03 (sept., 1H); 3,43 (s, 3H); 3,45 (m, 4H); 3,92 (q, 2H); 6,9-7,3 (m, 4H).

Beispiel 43

4-(4-Fluorphenyl)-3-hydroxymethyl-2-isopropyl-6-piperidino-pyridin-5-carbonsäureethylester

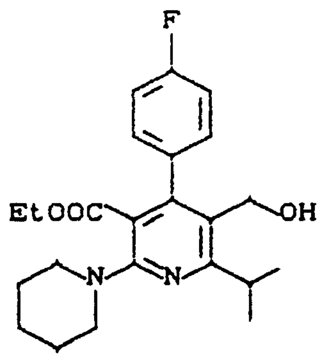

Die Herstellung erfolgt analog Beispiel 7.
$^1$H-NMR ($CDCl_3$): δ (ppm) = 0,98 (t, 3H); 1,28 (d, 6H); 1,62 (m, 6H); 3,42 (m, 5H); 3,90 (q, 2H); 4,32 (bs, 2H); 7,0-7,3 (m, 4H).

Beispiel 44

Methyl-erythro-(E)-7-[4-(4-fluorphenyl)-2-isopropyl-5-methoxymethyl-6-piperidino-pyrid-3-yl]-3,5-dihydroxy-hept-6-enoat

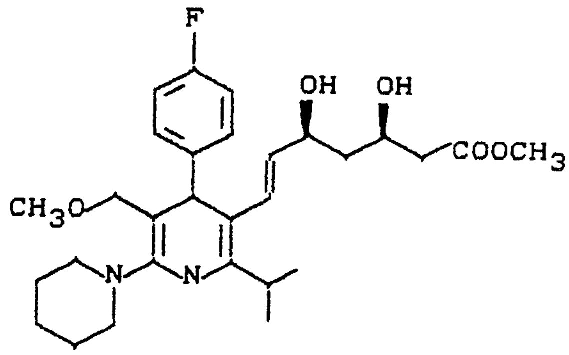

Aus der Verbindung aus Beispiel 43 wurde in Analogie zu den Reaktionen aus den Beispielen 19, 20, 21, 22, 9, 10, 11, 12, das Beispiel 44 hergestellt.

Beispiel 45

Methyl-erythro-(E)-7-[4-(4-fluorphenyl)-2-isopropyl-3-methoxymethyl-6-piperidino-pyrid-5-yl]-3,5-dihydroxy-hept-6-enoat

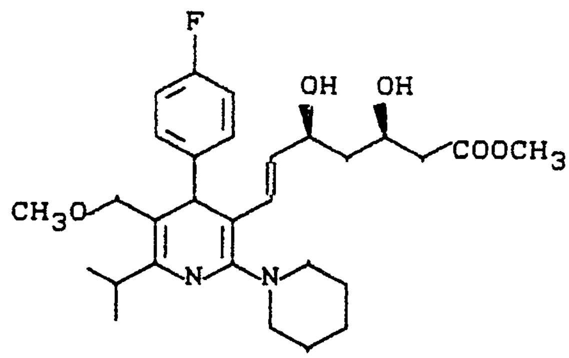

Aus der Verbindung aus Beispiel 43 wurde, in Analogie zu den Reaktionen aus den Beispielen 13, 14, 9, 10, 11, 12, das Beispiel 45 hergestellt.

Beispiel 46

Methyl-erythro-(E)-7-[2-dimethylamino-4-(4-fluorphenyl)-3-hydroxymethyl-6-isopropyl-pyrid-5-yl]-3,5-dihydroxy-hept-6-enoat

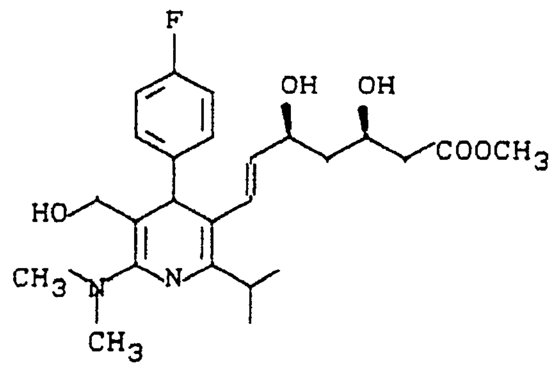

Beispiel 47

Methyl-erythro-(E)-7-[2-cyclopropyl-6-dimethylamino-4-(4-fluorphenyl)-5-methoxymethyl-pyrid-3-yl]-3,5-dihydroxy-hept-6-enoat

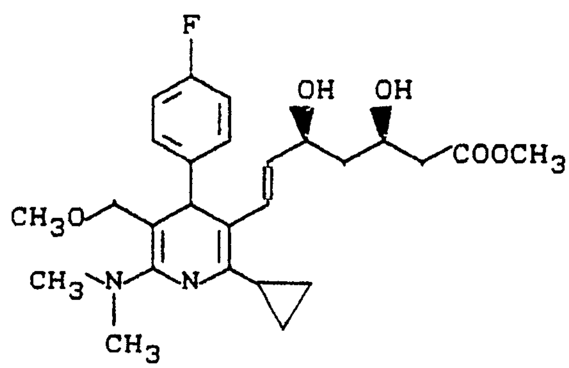

Beispiel 48

Methyl-erythro-(E)-7-[2-cyclopropyl-6-dimethylamino-4-(4-fluorphenyl)-5-hydroxymethyl-pyrid-3-yl]-3,5-dihydroxy-hept-6-enoat

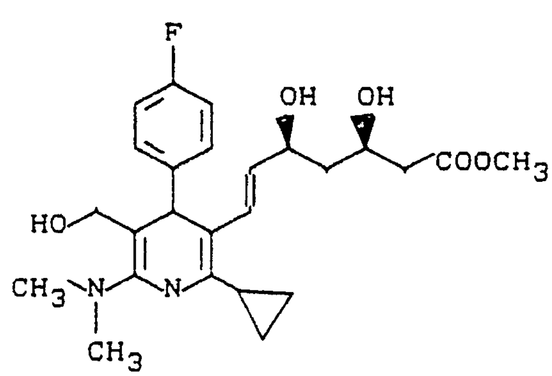

Beispiel 49

Methyl-erythro-(E)-7-[4-(4-fluorphenyl-3-hydroxymethyl-6-isopropyl-2-pyrrolidino-pyrid-5-yl]-3,5-dihydroxy-hept-6-enoat

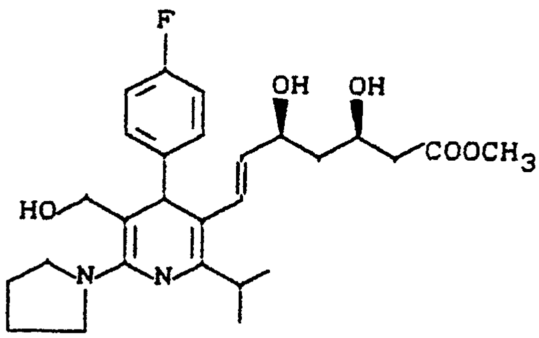

Beispiel 50

Methyl-erythro-(E)-7-[2-cyclopropyl-4-(4-fluorphenyl)-3-hydroxymethyl-6-pyrrolidino-pyrid-5-yl]-3,5-dihydroxy-hept-6-enoat

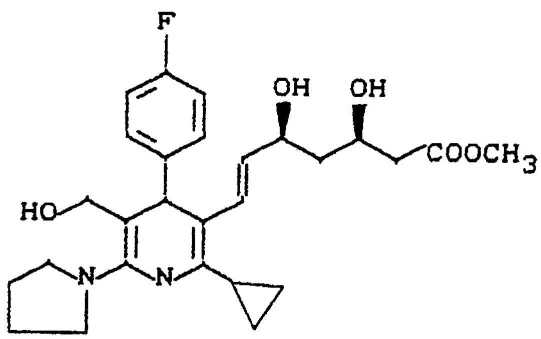

## Ansprüche

1. Substituierte Amino-pyridine der allgemeinen Formel

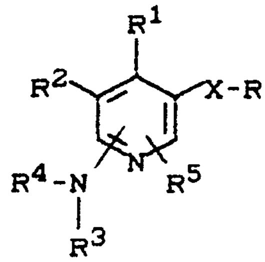

(I)

in welcher

X - für eine Gruppe der Formel $-CH_2-CH_2-$ oder $-CH=CH-$steht,

R - für eine Gruppe der Formel

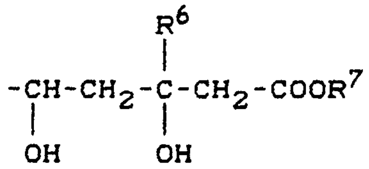

oder

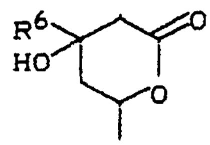

steht,
worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
$R^1$ - für Aryl mit 6 bis 10 Kohlenstoffatomen steht, das gegebenenfalls bis zu 4-fach gleich oder verschieden durch geradkettiges oder verzeigtes Alkyl, Alkylthio, Alkylsulfonyl, Alkoxy oder Alkoxycarbonyl mit jeweils bis zu 8 Kohlenstoffatomen substituiert ist, die ihrerseits durch Hydroxy oder Phenyl substituiert sein können, oder durch Aryl, Aryloxy, Arylthio mit 6 bis 10 Kohlenstoffatomen, Benzyloxy, Halogen, Nitro, Cyano, Trifluormethyl, Trifluormethoxy oder Benzyloxy substituiert ist,
$R^2$ - für eine Gruppe der Formel $-CH_2-OR^8$ oder -X-R steht,
worin
$R^8$ - Wasserstoff, geradkettiges oder verzweigtes Alkyl mit bis zu 10 Kohlenstoffatomen bedeutet, das gegebenenfalls durch Hydroxy, Halogen oder Phenyl substituiert ist,
- Aryl mit 6 bis 10 Kohlenstoffatomen bedeutet, oder
- eine Gruppe der Formel $-COR^9$ bedeutet,
worin
$R^8$ - geradkettiges oder verzweigtes Alkyl mit bis zu 8 Kohlenstoffatomen oder Phenyl bedeutet,
und X und R die oben angegebene Bedeutung haben,
$R^3$ und $R^4$ gleich oder verschieden sind und
- für Wasserstoff, geradkettiges oder verzweigtes Alkyl mit bis zu 8 Kohlenstoffatomen, Phenyl oder für eine Gruppe der Formel $A-R^{10}$ steht,
worin
A - Carbonyl oder Sulfonyl bedeutet
und
$R^{10}$ - Amino oder Alkoxy mit bis zu 8 Kohlenstoffatomen bedeutet, geradkettige oder verzweigtes Alkyl mit bis zu 8 Kohlenstoffatomen oder Phenyl bedeutet, die ihrerseits durch Hydroxy, Alkoxy mit bis zu 8 Kohlenstoffatomen oder Halogen substituiert sein können, oder
- Trifluormethyl bedeutet,
oder
$R^3$ und $R^4$ gemeinsam mit dem Stickstoffatom einen gesättigten oder ungesättigten 5- bis 7-gliedrigen Heterocyclus mit bis zu 3 Heteroatomen aus der Reihe Stickstoff, Schwefel oder Sauerstoff bilden,
und
$R^5$ - für geradkettiges oder verzweigtes Alkyl mit bis zu 10 Kohlenstoffatomen steht, oder
- für Cycloalkyl mit 3 bis 8 Kohlenstoffatomen steht
und deren Salze.

2. Substituierte Aminopyridine der Formel (I) nach Anspruch 1, worin
X - für eine Gruppe der Formel $-CH_2-CH_2-$ oder -CH = CH-steht,
R - für eine Gruppe der Formel

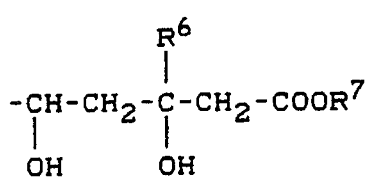

oder

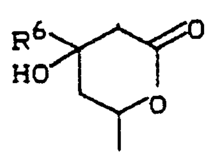

steht,

worin

$R^6$ - Wasserstoff oder geradkettiges oder verzweigtes Alkyl mit bis zu 8 Kohlenstoffatomen bedeutet

und

$R^7$- Wasserstoff oder geradkettiges oder verzweigtes Alkyl mit bis zu 8 Kohlenstoffatomen oder Benzyl bedeutet, oder

- Phenyl oder ein Kation bedeutet,

$R^1$ - für Phenyl steht, das gegebenenfalls bis zu 3-fach gleich oder verschieden durch geradkettiges oder verzweigtes Alkyl, Alkoxy oder Alkoxycarbonyl mit jeweils bis zu 6 Kohlenstoffatomen substituiert ist, die ihrerseits durch Phenyl substituiert sein können, oder durch Phenyl, Phenoxy, Fluor, Chlor, Brom, Benzyloxy, Trifluormethyl oder Trifluormethoxy substituiert ist,

$R^2$ - für eine Gruppe der Formel $-CH_2-OR^8$ oder X-R steht,

worin

$R^8$ - Wasserstoff, geradkettiges oder verzweigtes Alkyl mit bis zu 6 Kohlenstoffatomen bedeutet, das gegebenenfalls durch Hydroxy, Fluor, Chlor oder Phenyl substituiert ist, oder

- Phenyl bedeutet, oder

- eine Gruppe der Formel $-COR^9$ bedeutet,

worin

$R^9$ - geradkettiges oder verzweigtes Alkyl mit bis zu 6 Kohlenstoffatomen bedeutet,

und X und R die oben angegebene Bedeutung haben,

$R^3$ und $R^4$ gleich oder verschieden sind und - für Wasserstoff, geradkettiges oder verzweigtes Alkyl mit bis zu 6 Kohlenstoffatomen, Phenyl oder

- für die Gruppe der Formel $-A-R^{10}$ steht,

worin

A - Carbonyl oder Sulfonyl bedeutet

und

$R^{10}$ - geradkettiges oder verzweigtes Alkyl mit bis zu 6 Kohlenstoffatomen, Phenyl oder Trifluormethyl bedeutet

oder

$R^3$ und $R^4$ gemeinsam mit dem Stickstoffatom einen Morpholin-, Piperidin- oder Pyrrolidin ring bilden,

und

$R^5$ - für geradkettiges oder verzweigtes Alkyl mit bis zu 8 Kohlenstoffatomen, Cyclopropyl, Cyclopentyl oder Cyclohexyl steht

und deren Salze.

3. Substituierte Amino-pyridine der Formel (I) nach Anspruch 1, worin

X - für eine Gruppe -CH=CH- steht,

R - für eine Gruppe der Formel

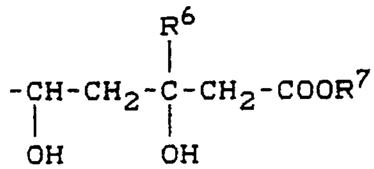

oder

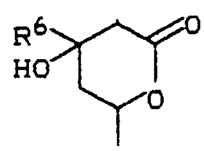

steht,

worin

$R^6$ - Wasserstoff, Methyl, Ethyl, Propyl, Isopropyl, Butyl, Isobutyl oder tert.Butyl bedeutet

und

$R^7$ - Wasserstoff, Methyl, Ethyl, Propyl, Isopropyl, Butyl, Isobutyl, tert.Butyl oder Benzyl bedeutet, oder

- ein Natrium-, Kalium-, Calcium-, magnesium-oder Ammoniumion bedeutet,

$R^1$ - für Phenyl steht, das gegebenenfalls bis zu 2-fach gleich oder verschieden durch geradkettiges oder verzweigtes Alkyl oder Alkoxy mit jeweils bis zu 4 Kohlenstoffatomen, Benzyl, Fluor, Chlor, Phenoxy oder Benzyloxy substituiert ist,

$R^2$- für eine Gruppe der Formel $-CH_2-OR^8$ oder -X-R steht,

worin

$R^8$ - Wasserstoff, geradkettiges oder verzweigtes Alkyl mit bis zu 4 Kohlenstoffatomen oder Phenyl bedeutet,

und X und R die oben angegebene Bedeutung haben,

$R^3$ und $R^4$ gleich oder verschieden sind und
- für Wasserstoff oder geradkettiges oder verzweigtes Alkyl mit bis zu 4 Kohlenstoffatomen oder Phenyl steht, oder
- für die Gruppe der Formel -A-$R^{10}$ steht,
worin
$R^{10}$ geradkettiges oder verzweigtes Alkyl mit bis zu 4 Kohlenstoffatomen bedeutet,
oder
$R^3$ und $R^4$ gemeinsam mit dem Stickstoffatom einen Piperidin- oder Pyrrolidinring bilden,
$R^5$ - für geradkettiges oder verzweigtes Alkyl mit bis zu 6 Kohlenstoffatomen oder Cyclopropyl steht,
und deren Salze.

4. Substituierte Amino-pyridine nach Anspruch 1 zur Bekämpfung von Krankheiten.

5. Verfahren zur Herstellung von substituierten Aminopyridinen der allgemeinen Formel

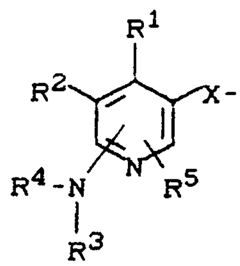

(I)

in welcher
X - für eine Gruppe der Formel $-CH_2-CH_2-$ oder $-CH=CH-$steht,
R - für eine Gruppe der Formel

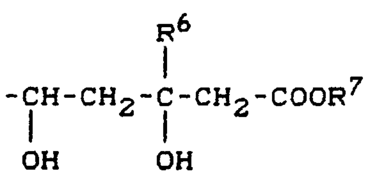 oder 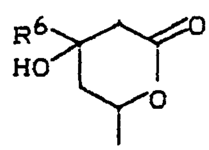

steht,
worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
$R^1$ - für Aryl mit 6 bis 10 Kohlenstoffatomen steht, das gegebenenfalls bis zu 4-fach gleich oder verschieden durch geradkettiges oder verzeigtes Alkyl, Alkylthio, Alkylsulfonyl, Alkoxy oder Alkoxycarbonyl mit jeweils bis zu 8 Kohlenstoffatomen substituiert ist, die ihrerseits durch Hydroxy oder Phenyl substituiert sein können, oder durch Aryl, Aryloxy, Arylthio mit 6 bis 10 Kohlenstoffatomen, Benzyloxy, Halogen, Nitro, Cyano, Trifluormethyl, Trifluormethoxy oder Benzyloxy substituiert ist,
$R^2$ - für eine Gruppe der Formel $-CH_2-OR^8$ oder -X-R steht,
worin
$R^8$ - Wasserstoff, geradkettiges oder verzweigtes Alkyl mit bis zu 10 Kohlenstoffatomen bedeutet, das gegebenenfalls durch Hydroxy, Halogen oder Phenyl substituiert ist,
- Aryl mit 6 bis 10 Kohlenstoffatomen bedeutet, oder
- eine Gruppe der Formel $-COR^9$ bedeutet,
worin
$R^9$ - geradkettiges oder verzweigtes Alkyl mit bis zu 8 Kohlenstoffatomen oder Phenyl bedeutet,
und X und R die oben angegebene Bedeutung haben,
$R^3$ und $R^4$ gleich oder verschieden sind und - für Wasserstoff, geradkettiges oder verzweigtes Alkyl mit bis zu 8 Kohlenstoffatomen, Phenyl oder für eine Gruppe der Formel A-$R^{10}$ steht,
worin

A - Carbonyl oder Sulfonyl bedeutet
und
$R^{10}$ - Amino oder Alkoxy mit bis zu 8 Kohlenstoffatomen bedeutet, geradkettige oder verzweigtes Alkyl mit bis zu 8 Kohlenstoffatomen oder Phenyl bedeutet, die ihrerseits durch Hydroxy, Alkoxy mit bis zu 8 Kohlenstoffatomen oder Halogen substituiert sein können, oder
- Trifluormethyl bedeutet,
oder
$R^3$ und $R^4$ gemeinsam mit dem Stickstoffatom einen gesättigten oder ungesättigten 5- bis 7-gliedrigen Heterocyclus mit bis zu 3 Heteroatomen aus der Reihe Stickstoff, Schwefel oder Sauerstoff bilden,
und
$R^5$ - für geradkettiges oder verzweigtes Alkyl mit bis zu 10 Kohlenstoffatomen steht, oder
- für Cycloalkyl mit 3 bis 8 Kohlenstoffatomen steht
und deren Salze, dadurch gekennzeichnet, daß man Ketone der allgemeinen Formel (VIII)

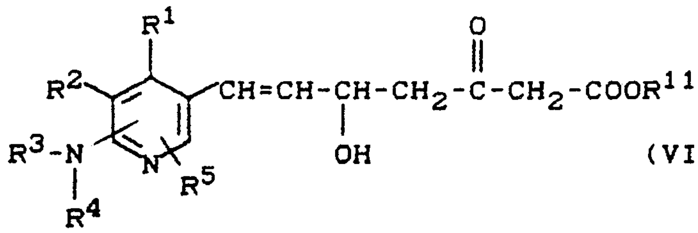

(VIII)

in welcher
$R^1$, $R^2$, $R^3$, $R^4$ und $R^5$ die oben angegebene Bedeutung haben,
und
$R^{11}$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = $-CH=CH-$) nach üblichen Methoden hydriert,
und gegebenenfalls Isomeren trennt.

6. Arzneimittel enthaltend mindestens ein substituiertes Amino-pyridin nach Anspruch 1.

7. Verfahren zur Herstellung eines Arzneimitteln nach Anspruch 6, dadurch gekennzeichnet, das man substituierte Amino-pyridine nach Anspruch 1 gegebenenfalls mit Hilfe von üblichen Hilfs- und Trägerstoffen in eine geeignete Applikationsform bringt.

8. Verwendung von substituierten Amino-pyridinen nach Anspruch 1 zur Herstellung von Arzneimitteln.

9. Verwendung von substituierten Aminopyridinen nach Anspruch 1 zur Bekämpfung von Krankheiten.

10. Ketone der allgemeinen Formel

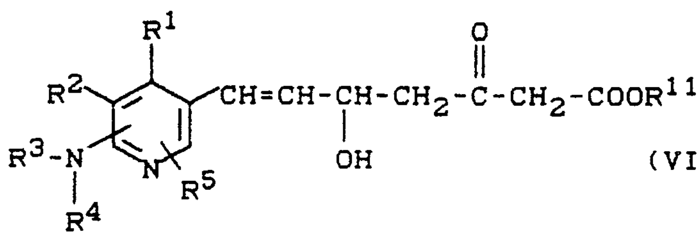

(VIII)

worin
$R^1$ - für Aryl mit 6 bis 10 Kohlenstoffatomen steht, das gegebenenfalls bis zu 4-fach gleich oder verschieden durch geradkettiges oder verzeigtes Alkyl, Alkylthio, Alkylsulfonyl, Alkoxy oder Alkoxycarbonyl mit jeweils bis zu 8 Koh lenstoffatomen substituiert ist, die ihrerseits durch Hydroxy oder Phenyl substituiert sein können, oder durch Aryl, Aryloxy, Arylthio mit 6 bis 10 Kohlenstoffatomen, Benzyloxy, Halogen, Nitro, Cyano, Trifluormethyl, Trifluormethoxy oder Benzyloxy substituiert ist,

$R^2$ - für eine Gruppe der Formel $-CH_2-OR^8$ oder -X-R steht,
worin
$R^8$ - Wasserstoff, geradkettiges oder verzweigtes Alkyl mit bis zu 10 Kohlenstoffatomen bedeutet, das gegebenenfalls durch Hydroxy, Halogen oder Phenyl substituiert ist,
- Aryl mit 6 bis 10 Kohlenstoffatomen bedeutet, oder
- eine Gruppe der Formel $-COR^9$ bedeutet,
worin
$R^9$ - geradkettiges oder verzweigtes Alkyl mit bis zu 8 Kohlenstoffatomen oder Phenyl bedeutet,
und
X - für eine Gruppe der Formel $-CH_2-CH_2-$ oder -CH = CH-steht,
R - für eine Gruppe der Formel

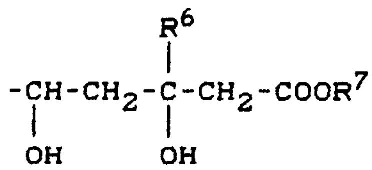 oder 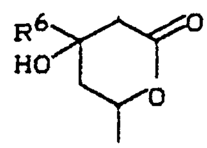

steht,
worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
$R^3$ und $R^4$ gleich oder verschieden sind und
- für Wasserstoff, geradkettiges oder verzweigtes Alkyl mit bis zu 8 Kohlenstoffatomen, Phenyl oder für eine Gruppe der Formel $A-R^{10}$ steht,
worin
A - Carbonyl oder Sulfonyl bedeutet
und
$R^{10}$ - Amino oder Alkoxy mit bis zu 8 Kohlenstoffatomen bedeutet, geradkettige oder verzweigtes Alkyl mit bis zu 8 Kohlenstoffatomen oder Phenyl bedeutet, die ihrerseits durch Hydroxy, Nitro, Cyano oder Halogen substituiert sein können, oder
- Trifluormethyl bedeutet,
oder
$R^3$ und $R^4$ gemeinsam mit dem Stickstoffatom einen gesättigten oder ungesättigten 5- bis 7-gliedrigen Heterocyclus mit bis zu 3 Heteroatomen aus der Reihe Stickstoff, Schwefel oder Sauerstoff bilden,
und
$R^5$ - für geradkettiges oder verzweigtes Alkyl mit bis zu 10 Kohlenstoffatomen steht, oder
- für Cycloalkyl mit 3 bis 8 Kohlenstoffatomen steht.
11. Verfahren zur Herstellung von Ketonen der allgemeinen Formel

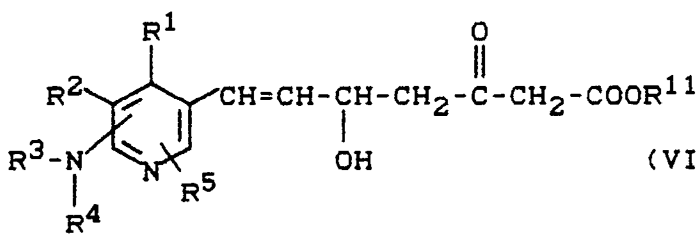

(VIII)

worin
$R^1$ - für Aryl mit 6 bis 10 Kohlenstoffatomen steht, das gegebenenfalls bis zu 4-fach gleich oder verschieden durch geradkettiges oder verzeigtes Alkyl, Alkylthio, Alkylsulfonyl, Alkoxy oder Alkoxycarbonyl mit jeweils bis zu 8 Kohlenstoffatomen substituiert ist, die ihrerseits durch Hydroxy oder Phenyl substituiert sein können, oder durch Aryl, Aryloxy, Arylthio mit 6 bis 10 Kohlenstoffatomen, Benzyloxy, Halogen, Nitro,

Cyano, Trifluormethyl, Trifluormethoxy oder Benzyloxy substituiert ist,
$R^2$ - für eine Gruppe der Formel $-CH_2-OR^8$ oder -X-R steht,
worin
$R^8$ - Wasserstoff, geradkettiges oder verzweigtes Alkyl mit bis zu 10 Kohlenstoffatomen bedeutet, das gegebenenfalls durch Hydroxy, Halogen oder Phenyl substituiert ist,
- Aryl mit 6 bis 10 Kohlenstoffatomen bedeutet, oder
- eine Gruppe der Formel $-COR^9$ bedeutet,
worin
$R^9$ - geradkettiges oder verzweigtes Alkyl mit bis zu 8 Kohlenstoffatomen oder Phenyl bedeutet,
und
X - für eine Gruppe der Formel $-CH_2-CH_2-$ oder -CH = CH-steht,
R - für eine Gruppe der Formel

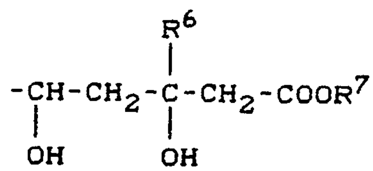
oder
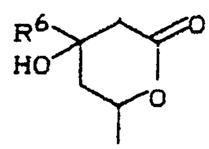

steht,
worin
$R^6$ - Wasserstoff oder geradkettiges oder verzweigtes Alkyl mit bis zu 10 Kohlenstoffatomen bedeutet
und
$R^7$ - Wasserstoff oder geradkettiges oder verzweigtes Alkyl mit bis zu 10 Kohlenstoffatomen bedeutet, das durch Phenyl substituiert sein kann, oder
- Aryl mit 6 bis 10 Kohlenstoffatomen oder ein Kation bedeutet,
$R^3$ und $R^4$ gleich oder verschieden sind und
- für Wasserstoff, geradkettiges oder verzweigtes Alkyl mit bis zu 8 Kohlenstoffatomen, Phenyl oder für eine Gruppe der Formel $A-R^{10}$ steht,
worin
A - Carbonyl oder Sulfonyl bedeutet
und
$R^{10}$ - Amino oder Alkoxy it bis zu 8 Kohlenstoffatomen bedeutet, geradkettige oder verzweigtes Alkyl mit bis zu 8 Kohlenstoffatomen oder Phenyl bedeutet, die ihrerseits durch Hydroxy, Nitro, Cyano oder Halogen substituiert sein können, oder
- Trifluormethyl bedeutet,
oder
$R^3$ und $R^4$ gemeinsam mit dem Stickstoffatom einen gesättigten oder ungesättigten 5- bis 7-gliedrigen Heterocyclus mit bis zu 3 Heteroatomen aus der Reihe Stickstoff, Schwefel oder Sauerstoff bilden,
hat,
und
$R^5$ - für geradkettiges oder verzweigtes Alkyl mit bis zu 10 Kohlenstoffatomen steht, oder
- für Cycloalkyl mit 3 bis 8 Kohlenstoffatomen steht,
dadurch gekennzeichnet, daß man Aldehyde der allgemeinen Formel (IX)

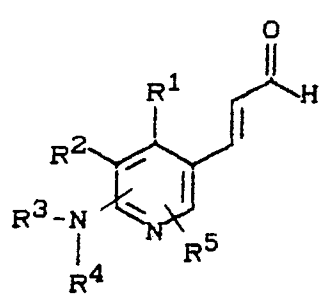
(IX)

in welcher

$R^1$, $R^2$, $R^3$, $R^4$ und $R^5$ die oben angegebene Bedeutung haben, in inerten Lösemitteln mit Acetessigester der allgemeinen Formel (X)

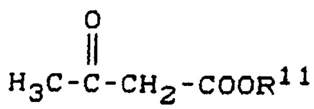

(X)

in welcher $R^{11}$ die oben angegebene Bedeutung hat, in Anwesenheit von Basen umsetzt.